# EUROPEAN PATENT SPECIFICATION

(11) **EP 0 314 690 B1**
(45) Date of publication and mention of the grant of the patent: **29.12.1993**
(21) Application number: 87904473.3
(22) Date of filing: 26.06.1987
(51) Int. Cl.: C07C 279/18

(54) **SIGMA BRAIN RECEPTOR LIGANDS AND THEIR USE**
SIGMA-GEHIRN-REZEPTOR-LIGANDEN UND DEREN VERWENDUNG
LIGANDS DE RECEPTEURS CEREBRAUX SIGMA ET UTILISATION DESDITS LIGANDS

(30) Priority: 10.07.1986 US 884150
(43) Date of publication of application: 10.05.1989
(73) Proprietor: STATE OF OREGON BY AND THROUGH OREGON STATE BOARD OF HIGHER EDUCATION ON BEHALF OF OREGON HEALTH SC. UNIV. AND UNIV. OF OREGON, Portland, Oregon 97201 (US)
(72) Inventor: WEBER, Eckard, Portland, OR 97202 (US); SONDERS, Mark, Portland, OR 97209 (US); KEANA, John, F., Eugene, OR 97405 (US)
(74) Representative: VOSSIUS & PARTNER
(86) International application number: PCT/US87/01545
(87) International publication number: WO 88/00583

(56) References cited:
- EP-A- 0 001 500
- EP-A- 0 035 374
- US-A- 4 169 154
- US-A- 4 575 514
- PROC. NATL. ACAD. SCI. USA, vol. 83, November 1986, pages 8784-8788;E. WEBER et al.: "1,3-Di(2-(5-3H) tolyl)guanidine: A selective ligand that labels omega-type receptors for psychotomimetic opiates and antipsychotic drugs"
- CHEMICAL ABSTRACTS, vol. 86, part 25, June 20, 1977, page 598, abstract 189787b, Columbus, Ohio, US; P.N. BHARGAVA et al.: "Synthesis of some new N, N'-di-(substituted 2-benzothiazolyl)guanidines as antituberculars and CNS depressants" & EGYPT. J. CHEM. 1974 (Pub. 1976), 17(6), 915 21
- CHEMICAL ABSTRACTS, vol. 86, part 17, April 25, 1977, pages 522, 523; abstract 12107h, Columbus, Ohio, US; A.A. STOLYARCHUK et al.: "Synthesis of furoylguanidines and comparison of their pharmacological properties with the properties of furoyl-ureas" & KHIM.-FARM. ZH 1976, 10(7), 72-7

## Description

This invention relates to the use of guanidine compounds which selectively bind sigma brain receptors for the preparation of pharmaceutical compositions useful in the diagnosis and treatment of mental illness, to novel N,N'-disubstituted compounds having such activity and utility and pharmaceutical compositions containing them and to methods for determining in vitro the sigma brain receptor binding activity of organic compounds,

A wide variety of substituted guanidines are disclosed in the patent literature. For example,
1,411,731 and 1,422,506 disclose diphenylguanidine as a rubber accelerator;
1,597,233 discloses N-o-tolyl-N'-phenyl-guanidine as a rubber accelerator;
1,672,431 discloses N,N'-di-o-methoxyphenylguanidine as being useful for therapeutic purposes, especially in the form of water-soluble salts;
1,730,338 discloses N-p-dimethyl-amino-phenyl-N'-phenylguanidine as a rubber accelerator;
1,795,738 discloses a process for the production of N,N'-dialkyl-di-substituted guanidines, including N-di-ethyl-N'-phenyl-guanidine, N-diethyl-N-isoamylguanidine, N-dimethyl-N'-isoamylguanidine and N-dimethyl-N'-ethylguanidine;
1,850,682 discloses a process for the preparation of disubstituted guanidine rubber accelerators bearing an additional substituent on the imine nitrogen atom;
2,145,214 discloses the use of disubstituted guanidines, e.g., diarylguanidines, especially dixylylguanidine, as parasiticides;
2,254,009 discloses sym-di-2-octyl-guanidine and 2,274,476 and 2,289,542 disclose sym-dicyclohexylguanidine as insectisides and moth larvae repellants;
2,633,474 discloses 1,3-bis(o-ethylphenyl)guanidine and 1,3-bis(p-ethylphenyl)guanidine as rubber accelerators;
3,117,994 discloses N,N',N''-trisubstituted guanidines and their salts as bacteriostatic compounds;
3,140,231 discloses N-methyl- and N-ethyl-N'-octylguanidines and their salts as antihypertensive agents;
3,252,816 discloses various N-substituted and unsubstituted cinnamyl-guanidines and generically the corresponding N'- and N''-alkyl substituted compounds and their salts as antihypertensive agents;
3,547,951 discloses 1,3-dioxolan-4-yl-alkyl-substituted guanidines which have anti-hypertensive activity and discloses lower alkyl, including n-butyl, as a possible substituent on the other amino group.

3,270,054 discloses N-2-adamant-1-yl- and N-2-homoadamant-1-yl-oxy-ethyl-thioethyl- and -aminoethyl-guanidine derivatives bearing at most two lower alkyl groups on the N'- and/or N''-nitrogen atom as sympathicolytic and anti-viral agents;
3,301,755 discloses N-ethylenically unsubstituted-alkyl-guanidines and the corresponding N'- and/or N''-lower alkyl compounds as hypoglycemic and antihypertensive agents;
3,409,669 discloses N-cyclohexylamino-(3,3-dialkyl-substituted-propyl)-guanidines and the corresponding N'-alkyl- and/or N''-alkyl-substituted compounds as hypotensive agents;
3,248,426 describes (Example 5) a 1,3-disubstituted guanidine whose substituents are hydrophobic hydrocarbon groups, one of which is napthylmethyl and the other is n-butyl;
3,639,477 discloses propoxyguanidine compounds as having anorectic properties;
3,804,898 discloses N-benzcyclobutenyl and N-benzycyclobutenyl-alkyl-guanidines and the corresponding N'-alkyl and/or N''-alkyl-substituted compounds as hypotensive agents;
3,968,243 discloses N-aralkyl substituted guanidines and the corresponding N'-alkyl-N''-alkyl and N',N'-aralkyl compounds as being useful in the treatment of cardiac arrhythmias;
3,975,533 discloses o-halo-bensylidene-amino-guanidines and their use as anti-depressants for overcoming psychic depression;
4,007,181 discloses various N,N'-disubstitued guanidines substituted on the imine nitrogen atom by a adamantyl as possessing antiarrhythmic and diuretic activities;
4,051,256 discloses N-phenyl- and N-pyridyl-N'-cycloalkyl-guanidines as antiviral agents;
4,109,014 discloses N-hydroxysubstituted guanidines and the corresponding N'-methyl disubstituted guanidines as vasoconstrictor agents;
4,169,154 discloses the use of guanidines in the treatment of depression;
4,393,077 discloses N-substituted and unsubstituted phenyl, N-substituted methyl-N'-unsubstituted, monosubstituted and disubstituted-N''-unsubstituted and substituted guanidines as ganglionic blocking agents; and
4,471,137 discloses N,N,N'N''-tetraalkyl guanidines as being sterically hindered bases useful in chemical synthesis.

For examples of other substituted guanidines, see, e.g., 1,422,506; 1,642,180; 3,159,676; 3,228,975; 3,248,426; 3,283,003; 3,320,229; 3,547,951; 3,639,477; 3,784,643; 3,975,533; 4,060,640; and 4,161,541.

Geluk, H.W., et al., J. Med. Chem., 12,712 (1969) describe the synthesis of a variety of adamantyl disubstituted guanidines as possible antiviral agents, including N,N'-di-(adamantan-1-yl)-guanidine hydrochloride, N-(adamantan-1-yl)-N'-cyclohexyl-guanidine hydrochloride and N-(adamantan-1-yl)-N'-benzyl-guanidine hydrochloride.

We have found that certain N,N'-disubstituted guanidines possess selective sigma receptor binding activity and thus may be useful for the preparation of a pharmaceutical composition useful in the diagnosis and treatment of psychosis.

Certain benzomorphan opiates, such as N-allylnormetazocine (SKF 10,047) and cyclazocine, in, addition to analgesia, cause hallucinations, depersonalization, drunkenness and other psychotomimetic effects in man. In monkeys, dogs and rodents the psychotomimetic opiates cause behavioral and autonomic effects that are unlike those observed with administration of classical opiates such as morphine or the opioid peptides. Specific sigma "opioid" receptors in the brain are believed to mediate such atypical effects. Martin et al, (1976) J. Pharmacol. Exp. Ther. 197, 517-532. The sigma receptors are believed to also mediate the psychotomimetic effects of phencyclidine [PCP, angel dust], or alternatively, that psychotomimetic opiates act at specifc PCP receptors. Zukin, R.S. & Zukin, S.R., (1981) Mol. Pharmacol. 20, 246-254; Shannon, H.E., (1983) J. Pharmacol. Exp. Ther. 225, 144-152; white, J.M. & Holtzman, S.G. (1983) Psychopharmacology 80, 1-9; and Zukin et al., (1986) J. Neurochem. 46, 1032-1041. PCP is a drug of abuse that causes a behavorial syndrome in man similar to that which is observed in schizophrenic psychosis. Aniline, O. & Pitts, F.N. Jr., (1982) CRC Critical Rev. Toxical. 10, 145-177. Because of the potent psychotomimetic effects of sigma opiates and PCP, it is believed that sigma (and/or PCP) receptors play a role in mental illness, particularly schizophrenia.

A systematic investigation of the role of sigma receptors in normal and abnormal brain function has been hindered by a lack of specific sigma receptor binding assays and bioassays. Development of such specific assays requires well-characterized, highly selective and potent sigma receptor ligands. Recent studies have shown that brain membrane receptors can be labeled in vitro with (+)[³H]SKF 10,047, Su, T.P., (1982) J. Pharmacol. Exp. Ther. 223, 284-290; (+)[³H] Ethylketazocine, Tam, S.W., (1983) Proc. Natl. Acad, Sci. U.S.A. 80, 6703-6707; or with (+)[³H]SKF 10,047, Tam, S.W. & Cook, L. (1984) Proc. Natl. Acad. Sci. U.S.A. 81, 5618-56721; Martin, et al., (1984) J. Pharmacol. Exp. Ther. 231, 539-544; and Mickelson, M.M. & Lahti, R.A. (1985) Res. Commun. Chem. Pathol. Pharmacol. 47, 255,263 although not selectively, Gundlach et al., (1985) Eur. J. Pharmacol. 113, 465-466; and Largent B.L., Gundlach, A.L. & Snyder, S.H. (1986) J. Pharmacol. Exp. Ther., (In Press), and with (+)[³H]3-(3-hydroxyphenyl)N-(1-propyl)piperidine ((+)[³H]3-PPP), Largent et al., (1984) Proc. Natl. Acad. Sci. U.S.A. 81, 4983-4987, which is apparently more selective for sigma receptors than the others.

After the initial in vitro studies by Martin and collaborators (1976), Keats and Telford, Keats, A.S. and Telford, J. "Analgesics: Clinical Aspects." In: Molecular Modification in Drug Design, R.F. Gould, ed., Advances in Chemistry Series #45 Amer. Chem. Soc., Wash. D.C. (1964), and Haertzen, Haertzen, C.A. Cyclazocine and Nalorphine on the Addiction Research Center Inventory (ARCI), Psychopharmacologia (Berl.) 18, 366-377 (1970), numerous investigators set out to biochemically characterize the different opiate receptors (mu receptors, kappa receptors and sigma receptors) in vitro.

The first evidence for the existence of a separate sigma receptor in test tube experiments was provided by Su(1982) in a paper describing an etorphine-inaccesible binding site in guinea pig brain membranes which was apparently selectively labeled by tritium labeled SKF-10,047. To overcome the fact that SKF-10,047 could label multiple opioid receptors in the brain, Su performed his receptor binding assay using tritium labeled SKF-10,047 in the presence of excess unlabeled etorphine. Etorphine is a very strong opiate agonist drug which is known to bind to delta receptors, mu receptors and kappa receptors with almost equal potency. Su used etorphine to saturate all mu, kappa and delta receptors in a brain membrane preparation and then added tritium labeled SKF-10,047. This enabled him to detect a sigma binding site that was apparently different from mu, kappa and delta receptors.

A major breakthrough in identifying the sigma receptor as a separate entity occured when Tam et al., (1984) demonstrated that the previous problems in selectively labeling the sigma receptor were caused by the fact that in all previous experiments a racemic SKF-10,047 preparation was used. Tam showed that using a tritium Labeled (+)-SKF-10,047 isomer he could selectively label a sigma receptor that was different from the mu, delta and kappa opioid receptors. On the other hand, Tam showed that (-)-SKF-10,047 apparently labeled the mu and kappa receptors but not the sigma receptors. Tam, S.W., Eur. J. Pharm. 109, 33-41 (1985). This finding has now been confirmed. (Martin et al, 1984). Moreover, there is evidance from behavorial experiments, Khazan et al., Neuropharm. 23, 983-987 (1984); Brady et al., Science 215, 178-180 (1981), that it is the (+)-SKF-10,047 isomer that is solely responsible for the psychotomimetic effects of SKF-10,047.

One of the most important findings of the biochemical characterization of the sigma receptor has been that this receptor binds all synthetic opiate drugs that are known to have hallucinogenic and psychotomimetic effects. Opiates that do not have psychotomimetic effects in vivo do not bind to this receptor. Most importantly, it has been shown that besides hallucinogenic opiate drugs, the sigma receptor also binds many antipsychotic drugs that are used clinically to treat hallucinations in schizophrenic patients. (Tam and Cook, 1984). The initial observations with regards to antipsychotic drug binding to the sigma receptor (Su, 1982) were subsequently extensively confirmed and extended by Tam and Cook (1984), who also showed that when one used radioactively labeled haloperidol, one of the most potent antipsychotic drugs that is used clinically, about half of the binding sites in brain membrane preparations are actually sigma receptors whereas the other half of the binding sites are apparently dopamine receptors. It has long been known that most antipsychotic drugs are also dopamine receptor antagonists and previously the beneficial actions of antipsychotic drugs in psychotic patients have been attributed to the dopamine receptor blocking effect of these drugs. It is clear from the work by Tam, however, that numermous clinically used psychotic drugs also bind to the sigma site. Therefore, all antipsychotic drugs that bind to the sigma receptor may in one way or another cause the beneficial effects of alleviating hallucinations. Taken together all these observations make the sigma receptor a prime candidate to be involved in the pathogenesis of mental illness, particularly schizophrenia in which hallucinations are a major clinical symptom.

The antipsychotic and anti-schizophrenic drugs that are currently in use have very strong side effects that are mainly due to their action on dopamine receptors. The side effects often involve irreversible damage to the extrapyramidal nervous system which controls movement functions of the brain. Patients under long term anti-schizophrenic drug treatment often develop a syndrome that involves permanent damage of their ability to control coordinated movement.

We have identified a novel class of compounds which bind to the sigma receptor.

The foregoing studies have shown that the sigma binding site has the characteristics of 1) stereoselectivity towards dextrorotatory benzomorphan opiates and insensitivity for naloxone; 2) high affinity for haloperidol and moderate to high affinity for phenothiazine anti-psychotic drugs which are also known to be potent dopamine receptor blockers; and 3) insensitivity for dopamine and apomorphine. This intriguing drug selectivity profile calls for a thorough analysis of the role of sigma receptors in normal and abnormal brain function. In order to do so, it is essential that a spectrum of highly selective and potent sigma receptor active compounds be available. This invention provides such compounds and methods for identifying other drugs having such activity.

### Objects of the Invention

It is an object of this invention to provide a novel class of compounds which selectively bind to sigma receptor sites and thus may be used for the preparation of a pharmaceutical composition useful in the diagnosis and treatment of psychosis

It is another object to provide such a compound which is radioactively tagged and which is useful for assaying in vitro the sigma receptor binding activity of organic compounds.

It is still another object to provide a method for determining the sigma receptor binding activity of organic compounds.

It is still another object to provide an in vitro screening method for assaying compounds having sigma receptor activity and utility as antipsychotic and antidepressant drugs.

It is still another object to provide a pharmaceutical composition which may be used in determining the relationship of abnormal psychotic-like behavior in a mammal displaying such behavior to sigma receptor system dysfunction.

Other objects will be apparent to those skilled in art to which this invention pertains.

In one aspect, this invention relates to tritium labeled N,N'-disubstituted guanidines of the formula 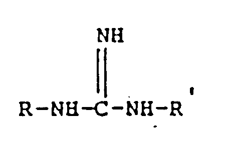
wherein R and R' each are adamantyl, cyclohexyl or a monocyclic carbocyclic aryl of at least 6 carbon atoms and wherein at least one of the ring carbon atoms of R and R' bears at least one tritium atom.

Preferred are compounds, wherein
a) one of R and R' is adamantyl and the other is 2-methylphenyl,
b) one of R and R' is adamantyl and the other is cyclohexyl,
c) both R and R' are adamantyl or both are cyclohexyl,
d) at least one of R and R' is azido-substituted carbocyclic aryl.

In another composition aspect, this invention relates to the use of guanidine derivatives for the preparation of a pharmaceutical composition, in unit dosage form and adapted for systemic administration to a human being, which comprises, per unit dosage, an amount effective to alter the sigma brain receptor-modulated activity of a human being displaying psychotic behavior or suffering from chronic depression, of an N,N'-disubstituted-guanidine in its water-soluble protonated form which displaces in vitro N,N'-di-(4-[³H]-2-methylphenyl)-guanidine bound to isolated mammalian brain membrane.

In another aspect, this invention relates to N-(4-azido-2-methylphenyl)-N-(2-methylphenyl)-guanidine.

In another aspect this invention relates to N,N'-di-(4-halo-2-methylphenyl)-guanidine.

In a method aspect, this invention relates to a method of determining the sigma brain receptor binding activity of an organic compound which comprises the steps of:
a) contacting in an aqueous medium a known amount of isolated mammalian brain membrane which has psychotomimetic benzomorphan binding activity, with a mixture of (i) a tritium labeled N,N'-disubstituted guanidine which selectively binds sigma brain receptors, in a known amount capable of being bound to the sigma receptors of that brain membrane; and (ii) varying known amounts of a water soluble organic compound to be assayed for sigma receptor binding activity.
b) separating the brain membrane from any of the tritium labeled compound which is not bound to the brain membrane in step a);
c) determining, from the molar relationship of the proportion of bound tritium labeled compound which is separated in step b) to the molar amount of the organic compound employed in step a), the sigma receptor binding activity of that organic compound.

In another method aspect, this invention relates to an in vitro screening method for antagonists to hallucinogenic benzomorphans having sigma receptor binding activity which comprises contacting in vitro isolated mouse vas deferens whose contractions in response to electrical stimulation has been augmented by a compound having sigma receptor binding activity and determining any reversal of that augmentation.

In another method aspect, this invention relates to the use of a pharmaceutical composition for a determining the relationship of abnormal psychotic-like behavior in a mammal displaying such behavior to sigma receptor dysfunction, which comprises administering thereto a sigma brain receptor-modulating amount of a water-soluble N,N'-disubstituted-guanidine which displaces in vitro N-N'-di-(4-[³H]-2-methylphenyl)-guanidine bound to mammalian brain membrane, effective to alter the sigma brain receptor-modulated mental activity of that mammal.

In another aspect, this invention relates to the use of a water soluble N,N'-disubstituted-guanidine which is an antagonist to the sigma receptor binding activity of a hallucinogenic benzomorphan, and/or which displaces in vitro N,N'-di-(4-[³H]-2-methylphenyl)-guanidine bound to mammalian brain membrane, preferably a compound of Formula II for the preparation of a pharmaceutical composition for treating a human being suffering from chronic depression or a psychotic mental illness associated with hallucinations, e.g., schizophrenia, which comprises administering thereto.

We have discovered that the disubstituted. guanidines of this invention have sigma receptor binding activity, as evidenced by their ability to displace (+)-[³H]SKF 10,047 from guinea pig brain membrane binding sites. We have also found that an [³H]-labeled derivative of one of these compounds, viz., [³H]-1,3-di-ortho-tolyl-guanidine (N,N'-di-(4-[³H]-2-methylphenyl)-guanidine, ([³H]-DTG]), which has the formula: 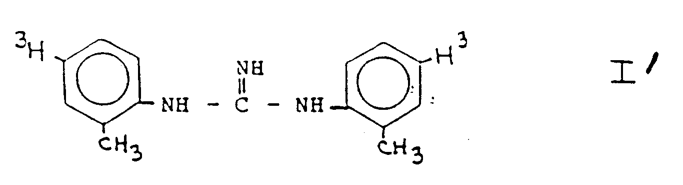
binds reversibly, saturably, selectively and with high affinity to sigma receptor binding sites in guinea pig brain membrane homogenates and slide-mounted rat and guinea pig brain sections. We have established that (+)-[³H]3-PPP binds to the same sites. Availability of the selective sigma ligands of this invention facilitates characterization of sigma receptors in vivo and in vitro.

The preferred N,N'-disubstituted guanidines of this invention are those of the formula I 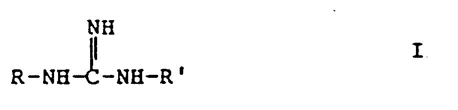
wherein R and R' each are an alkyl group of at least 4 carbon atoms or carbocyclic aryl groups of at least 6 carbon atoms, e.g., R and R', which can be the same or different, are alkyl of 4 of more carbon atoms, e.g., a 4 to 12, preferably a straight chain alkyl and more preferably a 4 to 8 carbon atom alkyl group, for example, butyl, isobutyl, tert-butyl, amyl, hexyl, octyl, nonyl and decyl; cycloalkyl of 3 to 12 carbon atoms, e.g., cyclopropyl, cyclopentyl, cyclohexyl, cycloheptyl, 1,4-methylene-cyclohexane, adamantyl, cyclopentylmethyl, cyclohexyl- methyl, 1- or 2-cyclohexylethyl and 1- 2- or 3-cyclo- hexylpropyl; carbocyclic aryl, alkaryl or aralkyl, e.g., of up to 18 carbon atoms and containing 1-3 separate or fused aromatic rings, e.g., phenyl, benzyl, 1- and 2-phenylethyl, 1-, 2-, or 3-phenyl- propyl; o-, m-, or p-tolyl, m,m'-dimethyl-phenyl, o-,m- or p,-ethylphenyl, m,m'-diethyl-phenyl, m-methyl-m'-ethyl-phenyl and o-propyl-phenyl, naphthyl, 2-naphthyl, and biphenyl.

Additionally, 1, 2, 3 or more substituents which are chemically and physiologically substantially inert compared to the guanidine group may be present on the R and R' hydrocarbon groups, e.g., alkyl of 1-8 carbon atoms, e.g., methyl, ethyl; halo, e.g., chloro, bromo, iodo, fluoro; nitro; azido, cyano; isocyanato; amino; lower-alkylamino; di-lower-alkylamino; trifluoromethyl; alkoxy of 1-8 carbon atoms, e.g., methoxy, ethoxy and propoxy; acyloxy, e.g., alkanoyloxy of 1-8 carbon atoms, e.g., acetoxy and benzoxy; amido, e.g., acetamido, N-ethylacetamido; carbamido, e.g., carbamyl, N-methylcarbamyl, N,N'-dimethylcarbamyl; etc.

Especially preferred are compounds of Formula II wherein R and R' each are phenyl groups, which need not necessarily be identical, substituted with one or more of the foregoing substituents, for example, in the o-, m- or p-position or the o-, p- or m-,m'-position, when the phenyl group is disubstituted, or R is as herein defined and R' is adamantyl. Specific examples are those wherein R and R' both are phenyl or p-tolyl; R is o-tolyl and R' is p-bromo-o-tolyl, p-CF₃-o-tolyl, p-iodo-o-tolyl, p-iodo-phenyl, p-azido-o-tolyl, cyclohexyl or adamantyl; and R is phenyl and R' is p-bromo-o-tolyl, p-iodo-o-tolyl, m-nitro-phenyl, or p-iodo-phenyl.

The present invention also relates to the use of these compounds for the preparation of a pharmaceutical composition in unit dosage form and adapted for systemic administration to a human being, which comprises, per unit dosage, an amount effective to alter the sigma brain receptor-modulated activity of a human being, of a water soluble N-N'-disubstituted guanidine which displaces in vitro N-N'-di (4-[³H]-2-methylphenyl)-guanidine bound to isolated mammalian brain membrane.
Preferably, the guanidine is a compound o the formula I 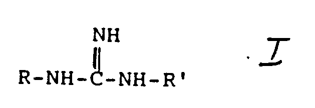
wherein R and R' each is an alkyl group of at least 4 carbon atoms, a cycloalkyl group of 3 to 12 carbon atoms or a carbocyclic aryl group of at least 6 carbon atoms. Especially preferred in the pharmaceutical composition are those guanidine compounds, wherein
a) at least one of R and R' is o-tolyl or adamantyl,
b) at least one of R and R' is o-tolyl,
c) R and R' are n-butyl,
d) R and R' are o-tolyl,
e) R and R' are phenyl,
f) R and R' are 2-methyl-4-bromo-phenyl,
g) R is o-tolyl and R' is p-azido-o-tolyl,
h) R is o-tolyl and R' is cyclohexyl,
i) R is o-tolyl and R' is adamantyl,
j) both R and R' are adamantyl and
k) R is adamantyl and R' is phenyl.

The highly active disubstituted guanidines of this invention, e.g., DTG, have substantially the same stereo-configuration as (+)3-PPP with phenyl axial and with (+)-SKF 10,047 with the piperidine ring in skew-boat form and N-allyl axial. This similarity in spacial configuration of compounds having sigma receptor binding activity provides a screening technique for predicting the probably level of sigma receptor binding activity of other N,N'-disubstituted guanidines.

Contemplated equivalents of the foregoing compounds wherein one or both of R and R' are carbocyclic aryl are those wherein the aryl group is heterocyclic, e.g., 2- and 4-pyridyl, 2- and 3-N-methyl-pyrrolyl, 2- and 3-furamyl, 2- and 3-thiofuranyl, 2- and 3-benzofuranyl, 2-benzoxazolyl, etc.

Examples of those compounds which have been isolated and/or prepared and found to possess the aforesaid in vitro [³H-] DTG displacement activity are N,N'-dibutyl-guanidine, N,N'-diphenyl-guanidine, N,N'-di-o-tolyl-guanidine, N,N'-(2-methyl-4-bromo-phenyl)-guanidine, and N,N'-di-(2-methyl-4-iodo-phenyl)-guanidine, N-(2-methyl-4-azido-phenyl)-N'-(2-methylphenyl)-guanidine, N-(adamantyl)-N'-(2-methylphenyl)-guanidine.

The level of sigma receptor activity of the disubstituted guanidines can also be determined in vivo in a discriminative stimulus property test employing rats trained to discriminate between intraperitoneal injections of cyclazocine (2.0 mg/kg) and saline in a discrete-trial avoidance paradigm with sessions of 20 trials each. For example, DTG and DPG were fully substitutable for cyclazocine at the same concentrations. (Holtzman, S.G., Emeroy University, Atlanta, Georgia, private communication).

The sigma receptor activity of compounds in vivo can also be determined by a screening procedure which comprises contacting in vitro isolated mouse vas deference whose contraction in response to electrical stimulation has been augmented by a compound, preferably a hallucinogenic sigma benzomorphan, e.g., (+)-PPP, with the test compound of various concentrations and determining (measuring) the amount, if any, of the reversal of that augmentation.

Although the discussion hereinafter of the experiments conducted by us relates to one of these selective sigma receptor ligands, viz., N,N'-di-o-tolyl-guanidine (DTG), the activity and utility of that compound apply comparably to the other disubstituted guanidines which compete with and displace in vitro N,N'-di-(4-[³H]-2-methylphenyl)-guanidine bound in vitro to isolated guinea pig brain nembrane.

In carrying out the sigma receptor binding activity measurement method of this invention, a known amount of a mammalian brain membrane, e.g., human or other primate porcine, rodent, e.g., rat or guinea pig, which has (+)-SKF 10,047 and like psychomimetic benzomorphan binding activity is contacted in a suitable aqueous vehicle, e.g., physiological saline solution, with a mixture, usually in a solution in a suitable aqueous vehicle of (i) a tritium labeled N,N'-disubstituted guanidine of this invention having sigma receptor binding activity, in an amount capable of being fully bound to the abovesaid amount of membrane and (ii) a water soluble organic compound whose sigma receptor activity is to be assayed, in known amounts, sufficiently varied to obtain a dose-response curve. The techniques for obtaining a dose-response curve are standard and well known to those skilled in the art. Typically, one could employ molar amounts varying as much as from 10⁻³ to 10³ of the molar amount of the tritium labeled compound present in the mixture, e.g., employing from 10 to 120 and preferably from 30 to 90 such mixtures.

If the organic compound being assayed has sigma receptor binding activity, a portion of the tritium labeled compound which, in the absence of the organic compound would bind to the membrane remains unbound and is thus separable from the membrane. The amount which remains unbound is proportional to the sigma receptor binding activity of the organic compound and the molar ratio thereof in the mixture to the tritium labeled compound.

The two compounds can be employed at any convenient collective concentration, e.g., from 10⁻⁸ to 10³ mM.

In the next step, the membrane is separated from and washed until free of the solution in which step (a) is conducted. In the next step, the amount of tritium labeled compound which is thus separated from the membrane is determined, e.g., by measuring the collective radio-activity-level of the separated solution and wash water and comparing that radioactivity to that obtained when the foregoing steps are conducted with the same amount of tritium labeled N,N'-disubstituted guanidine in the absence of the organic compound.

In the next step of the method, the activity of sigma receptor binding activity of the organic compound is determined from the dose response curve thus obtained.

Preferred is a method, wherein the brain membrane is porcine, rat, human or guinea pig.

Further preferred is a method, wherein the organic compound is a N,N'-disubstituted guanidine of the formula 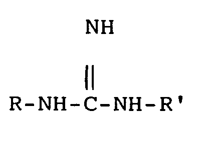
wherein R and R' each is an alkyl group of at least 4 carbon atoms or a carbocyclic aryl group of at least 6 carbon atoms.

All of the foregoing steps are conventional and have been employed in the prior art with other types of 3H-labeled compounds having sigma receptor binding activity. The method of this invention is, however, unique in that the tritium labeled N,N'-disubstituted guanidines of this invention are highly selective to binding by the sigma receptors and therefore will not compete with organic compounds which bind to other brain receptors.

These disubstituted guanidines can readily be prepared by conventional chemical reactions, e.g., when R and R' are the same, by reaction of the corresponding amine with cyanogen bromide. Other methods which can be employed includes the reaction of an amine with a preformed alkyl or allyl cyanamide. See Safer, S.R. et al., J. Org. Chem., 13:924 (1948). This is the method of choice in our laboratory for producing 1,3-disubstituted guanidines in which the substituents are not identical. For a recent synthesis of unsymmetrical guanidines, see G.J. Durant et al., J. Med. Chem., 28:1414 (1985), and C.A. Maryanoff et al., J. Org. Chem., 51:1882 (1986).

The characterization of sigma receptors in vitro has been difficult because of the lack of selective drug ligands. Most benzomorphan opiates crossreact with other (mu, delta, kappa), opioid receptors and are therefore of only limited value for characterizing and isolating receptors. Pasternak et al.; (1981) J. Pharmacol. Exp. Ther. 219, 192-198; Zukin, R.S. & Zukin, S.R., (1981) Mol. Pharm. 20, 246-254; and Tam, S.W., (1985) Eur. J. Pharmacol. 109, 33-41. [³H]DTG, binds specifically and with high affinity to a single class of binding sites in guinea pig brain membranes. The binding characteristics and the drug specificity profile of these sites are concordant with those proposed for the sigma receptor, including 1) naloxone insensitivity and stereoselectlvity for dextrorotatory isomers of benzomorphan opiates such as (+)SKF 10,047, (+)cyclazocine and (+)pentazocine; 2) high affinity for haloperidol and certain phenothiazine antipsychotic drugs; 3) stereoselectivity for (-)butaclamol; and (4) insensitivity to dopamine and apomorphine. [³H]-DTG is one of only two known compounds that are selective for the sigma site. The other, (+)[³H]3-PPP, originally proposed to be a dopamine autoreceptor agonist, has recently been shown to be selective for sigma sites in rat brain membrane binding assays. Largent et al., (1984), supra. Our experiments confirm these findings in the guinea pig and shown that [³H]DTG and (+)[³H]3-PPP have virtually identical receptor binding characteristics and drug selectivity profiles. Previous studies have shown that sigma sites can also be labeled with (±)[³H]SKF 10,047, (+)[³H]ethylketazocine and with (+)[³H]SKF 10,047. However, these ligands are not selective for the sigma site and require the presence of appropriate drugs in the binding assays to mask crossreacting non-sigma binding sites.

[³H]DTG has a number of advantages as a sigma ligand. It is highly selective for the sigma site (unlike [³H]SKF 10,047 and (+)[³H]Ethylketazocine), it has a high degree of specific binding (90-97% of total binding) and it has a relatively simple chemical structure that is not chiral (unlike (+)[³H]3-PPP and the benzomorphan opiates). These characteristics make it a good starting compound for the synthesis of analogs for structure-activity studies and for the design of irreversible sigma receptor ligands, e.g., compounds of Formula II wherein at least one of R and R' is azido-substituted carbocyclic aryl.

The sigma site labeled with [³H]DTG is clearly not related to conventional (mu, delta, kappa) opioid receptors as it is naloxone insensitive and shows stereoselectivity for dextrorotatory isomers of benzomorphan drugs. This is a reversed stereoselectivity compared to naloxone-sensitive opioid receptors which are selective for levorotatory isomers of opiates. Sigma receptors should therefore not be referred to as sigma "opioid" receptors. The drug selectivity of sigma sites for dextrorotatory isomers of psychotomimetic opiates does, however, correlate well with the pharmacological profile of dextrorotatory versus levorotatory opiates in animal tests designed to differentiate between conventional opioid receptor activity and sigma (behavioral) activity of benzmorphan drugs. Cowan, A. (1981) Life Sci. 28, 1559-1570; Brady, K.T. et al, (1982) Science 215, 178-180; and Khazan, N. et al (1984) Neuropharmacol. 23, 983-987.

Autoradiography studies using [³H]DTG visualize the sigma site in slide-mounted rodent brain sections and confirm that sigma sites are different from mu, delta, and kappa opioid receptors as the distribution of [³H]DTG binding is rather distinct from the distribution of mu, delta, kappa opioid receptors. The anatomical distribution of [³H]DTG binding sites is, however, very similar if not identical to the distribution of (+)[³H]3-PPP binding sites, further confirming that the two radioligands label identical binding sites. The high affinity of the [³H]DTG binding site for haloperidol and for certain phenothiazine antipsychotics (TABLE I) which are also dopamine D₂ receptor antagonists raises the question as to the relation of sigma receptors to dopamine D₂ receptors. The results presented show that the [³H]DTG site is clearly distinct from dopamine D₂ receptors, because the autoradiographic distribution of dopamine receptor is dissimilar and because dopamine and apomorphine do not interact with the [³H]DTG binding site.

Furthermore the sigma site labeled with [³H] is stereoselective for (-)butaclamol which is a reversed stereoselectivity compared to the dopamine D₂ receptors which are stereoselective for (+)butaclamol.

The haloperidol-sensitive sigma site labeled with [³H]DTG was found to have a moderate affinity for the potent hallucinogen PCP in competition experiments. This is in agreement with findings by others who used (+)[³H]SKF 10,047, [³H](+)SKF 10,047 or (+)-[³H]3-PPP to label sigma sites. In PCP receptor binding assays, however, [³H]-PCP labeled predominantly (but not exclusively) a haloperidol-insensitive PCP binding site, termed PCP/sigma opiate receptor by Zukin and colleagues, Zukin et al., (1981, 1986) supra, which is separate from the haloperidol-sensitive sigma site labeled with [³H]DTG or (+)[³H]3-PPP. In contrast, [³H]DTG appears to label exclusively the haloperidol sensitive sigma site, since all specific binding is displacable by haloperidol and the anatomical distribution of [³H]DTG binding is distinct from the distribution of PCP receptors. Furthermore, unlabeled DTG is virtually inactive in a [³H]-PCP binding assay (S. William Tam, E.I. DuPont De Nemours & Co., Wilmington, Delaware, personal communication). There is some controversy as to which of the two binding sites is responsible for causing the behavioral effects of PCP and psychotomimetic benzomorphan opiates and would therefore correspond to the sigma receptor postulated by Martin et al., (1976), supra. Zukin and his collaborators have argued that the behavioral effects of both PCP and psychotomimetic benzomorphan opiates are mediated by the haloperidol-insensitive PCP site, to which benzomorphan opiates bind with moderate affinity. Largent et al., (1986), supra, cited circumstantial evidence suggesting that it is equally likely that the behavioral effects of both PCP and psychotomimetic opiates are mediated through the haloperidol-sensitive sigma site. As [³H]DTG exclusively labels the haloperidol sensitive sigma site and does not interact significantly with the haloperidol-insensitive PCP site, behavioral studies using DTG or other symmetrically substituted guanidines of this invention as prototypical sigma ligands should resolve this issue.

Perhaps the most important aspect of the findings on the drug specificity of sigma sites that have emerged from this and other studies is that they interact with certain very potent hallucinogenic drugs (haloperidol, phenothiazines) that are used clinically to treat schizophrenia. This intriguing drug selectivity profile facilitates studies aimed at investigating the role of sigma receptors in antipsychotic drug action and abnormal brain function. The availability of DTG and like N,N'-disubstituted guanidines as a selective sigma ligand should serve to facilitate such studies.

Like guanidines generally and N,N'-diphenyl-guanidine specifically, the disubstituted guanidines of this invention, including those of formula II, are accelerators for the vulcanization of rubbers, e.g., natural rubbers and epoxy group-containing acrylic rubber, and can be used for such purpose in the same manner as N,N'-diphenylguanidine. Thus [H³]-DTG can be incorporated into a vulcanized rubber object, e.g., a tire tread, and rate of loss of rubber therefrom by water can be monitored by rate of loss of radioactivity.

The compounds of this invention have highly selective affinity for sigma receptor. Consequently, they may have some of the activities of the benzomorphans, i.e., those produced by binding to the haloperidol-sensitive sigma receptor but not those produced by the binding of benzomorphans to other non-sigma receptors. For instance, benzomorphans may act at sigma receptors to cause mydriasis and tachycardia and their pronounced psychotomimetic effects. DTG is therefore an effective tool to demonstrate the physiological effects mediated by the sigma receptor which, to date, have been obscurred by cross-reactivity of benzomorphans with non-sigma receptors. Additionally, at least some of the compounds of Formula II, e.g., N,N'-diphenyl and N,N'-di-o-tolyl-guanidine, are antagonists on the sigma receptors in the nerve terminals in the mouse vas deferens, were sigma receptors stimulate nor-adrenaline release (a phenomena discovered by us and which provides a new screening test for CNS-stimulants and depressants), and thus are blood pressure lowering (antihypertensive) agents. Other compounds of Formula II are agonists and thus increase blood pressure. Those which are antagonists to the sigma receptor binding activity, e.g., on the sigma sites of the mouse as deferens, of hallucinogenic benzomorphans, e.g., (+)3-PPP and TCP; are useful in the amelioration of the symptoms of a psychotic mental illness associated with the hallucinations, e.g., schizophrenia.

In carrying out the method of treatment aspect of this invention, e.g., treating a human being suffering from a psychotic mental illness associated with hallucinations there is administered thereto a water-soluble N,N'-disubstituted guanidine which is an antagonist to the sigma receptor binding activity of a hallucinogenic benzomorphan in an amount effective to ameliorate the hallucinations. Preferably, the guanidine is a compound of Formula II wherein R and R' each is the same and each is an alkyl group of at least 4 carbon atoms, a cycloalkyl group of 3 to 12 carbon atoms or a carbocyclic aryl group of at least 6 carbon atoms. In a preferred aspect, the human being is schizophrenic; in another preferred aspect, the compound is N,N'-di-(2-methylphenyl)-guanidine, N-(adamantyl)-N'-(cyclohexyl)-guanidine, N-adamantyl-N'-(2-methylphenyl)-guanidine, N-cyclohexyl-N'-(2-methylphenyl)-guanidine, N,N'-di-(2-cyclohexyl)-guanidine or N,N'-di-(2-adamantyl)-guanidine.

N,N'-disubstituted guanidines, e.g., of Formula I, can act in an agonistic, antagonistic or inverse agonistic manner in relation to the prototypical sigma benzomorphans. Those which act as antagonists can therefore be expected to affect pupil size, heart rate and mentation in a direction opposite that caused by benzomorphans which can be determined by standard tests in laboratory animals. The type and level of activity for a given dosage of each compound can be conventionally determined by routine experimentation using well known pharmacological protocols for each of the activities; the corresponding indications treatable at that dosage will be well known to skilled workers based on the pharmacological results. The compounds of this invention are particularly noteworthy for their antipsychotic activity to treat psychotic conditions, e.g., schizophrenia, by analogy to the known agents prolixin and Thorazine and for diagnosing sigma receptor intoxicated conditions.

The ³H-DTG of this invention is useful as a screening tool for compounds, such as the disubstituted guanidines of this invention, which are selective ligands for the sigma receptor binding site. As such, they are useful for screening for compounds useful for the diagnosis and treatment of sigma receptor mediated hallucinogenic mental disorders. For example, such a compound which is an agonist to a putative natural ligand will temporarily exacerbate such a mental disorder which is the result of an overabundance of the endogenous ligand and will ameliorate a mental disorder which is the result of an abnormal insufficiency of the natural ligand. The converse occurs when the disubstituted guanidine is an antagonist to the putative endogenous ligand. In either case, the temporary alteration of the mental disorder by the administered ligand confirms that it is a sigma receptor associated disease, thereby eliminating other possible causes thereof, e.g., chemical toxicity, and facilitating the treatment thereof.

[³H]-DTG also binds to human brain membrane receptors with high affinity as determined in our laboratory. Therefore another use of [³H]-DTG is to explore the neurochemistry of mental disease by measuring the fluctations in receptor density or function in post-mortem tissue of patients manifesting psycho- or neuropathology as contrasted with tissue from normals (unaffected controls). This topic can be studied by both receptor binding assays and autoradiography.

The compounds of this invention can be administered orally or by injection, e.g., intramuscular, intraperitoneal or intravenously. The optimal dose can be determined by conventional means. Because most if not all of the disubstituted guanidines employed in this invention are substantially water insoluble, they are ordinarily administered in their protonated form, e.g., as a pharmaceutically acceptable salt of an organic or inorganic acid, e.g., hydrochloride, sulfate, hemi-sulfate, phosphate, nitrate, acetate, oxalate, citrate, etc.

The compounds of this invention can be employed in mixture with conventional excipients, i.e., pharmaceutically acceptable organic or inorganic carrier substances suitable for parenteral, enteral or topical application which do not deleteriously react with the active compounds. Suitable pharmaceutically acceptable carriers include but are not limited to water, salt solutions, alcohols, vegetable oils, polyethylene glycols, gelatin, lactose, amylose, magnesium stearate, talc, silicic acid, viscous paraffin, perfume oil, fatty acid monoglycerides and diglycerides, pentaerythritol fatty acid esters, hydroxy methylcellulose, polyvinylpyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

For parenteral application, particularly suitable are solutions, preferably oily or aqueous solutions, as well as suspensions, emulsions, or implants, including suppositories. Ampules are convenient unit dosages.

For enteral application, particularly suitable are tablets, dragees, or capsules having talc and/or a carbohydrate carrier or binder or the like, the carrier preferably being lactose and/or corn starch and/or potato starch. A syrup, elixir or the like can be used wherein a sweetened vehicle is employed. Sustained release compositions can be formulated including those wherein the active compound is protected with differentially degradable coatings, e.g., by microencapsulation, multiple coatings, etc.

Generally, the compounds of this invention are dispensed in unit dosage form comprising about 0.1 to about 10³ mM of the N,N'-disubstituted guanidine per each unit dosage.

Parenteral administration, e.g., i.p. or i.m., is preferred, the compounds of this invention being particularly valuable in the treatment of humans afflicted with a psychotic disease, e.g., schizophrenia. In this regard, they can be employed in substantially the same manner as the known major tranquilizers.

It will be appreciated that the actual preferred amounts of active compounds used will vary according to the specific compound being utilized, the particular compositions formulated, the mode of application, and the particular site of administration. Optimal administration rates for a given protocol of administration can be readily ascertained by those skilled in the art using conventional dosage determination tests conducted with regard to the foregoing guidelines.

Thus, the invention relates to a pharmaceutical composition, in unit dosage form and adapted for systemic administration to a human being, which comprises, per unit dosage, an amount effective to alter the sigma brain receptor-modulated activity of a human being, of a water soluble N,N'-disubstituted guanidine which displaces in vitro N,N'-di (4-[³H]-2-methylphenyl)-guanidine bound to isolated mammalian brain membrane.

In a preferred embodiment this pharmaceutical composition is adapted for oral administration.

In a further preferred embodiment the pharmaceutical composition is adapted for parenteral injection. Preferably, the pharmaceutical composition contains from about 0,1 mg to about 1 g of the disubstituted guanidines per unit dosage.

The sigma receptor binding activity of N,N'-di-o-tolyl-guanidine (DTG) was discovered during studies on the purification and characterization of an endogenous sigma receptor ligand. In the initial phases of this work numerous extraction solvents were tested for their suitability to extract endogenous sigma receptor ligand from cow brains. Certain extraction solvents (particularly acetone) contained an unidentified material that potently displaced (+)-[³H]SKF 10,047 from guinea pig brain membrane binding sites. A receptor binding characterization of the material revealed that the binding activity was competitive, reversible and rather potent. Because of the potent sigma receptor binding properties of three compounds that were found in these solvents, these chemicals were purified to homogeneity using various reverse phase HPLC procedures and their structure determined. Their structure was determined by a combination of different methods including high resolution mass spectroscopy, nuclear magnetic resonance and UV-spectrophotometry. One of the three sigma receptor active chemicals that were purified from the extraction solvents proved to be 1,3-di-o-tolyl-guanidine (DTG).

After its structural characterization, synthetic DTG was tested in the (+)-[³H]SKF 10,047 binding assay and was found to displace (+)-[³H]SKF 10,047 from its brain membrane binding sites with a K_{d} of 70 nM. The displacement was competitive and fully reversible.

The other two compounds that were isolated and characterized were di-phenyl-guanidine (DPG) and di-butyl-guanidine (DGB), both of which were active in the (+)-[³H]SKF 10,047 binding assay.

Because of the high potency of DTG and related di-substituted guanidines to displace (+)-[³H]-SKF 10,047 from its binding sites it was decided to synthesize a tritium labeled derivative of DTG. To do so, N,N'-di-(2-methyl-4-bromo-phenyl)-guanidine was subjected to catalytic reduction with tritium gas, which replaced the two bromine atoms with tritium atoms to produce N,N'-di-(p-[³H]-o-tolyl)-guanidine, as described below.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the preceding text and the following,examples, all temperatures are set forth uncorrected in degrees Celsius and all parts and percentages are by weight, unless otherwise indicated.

### Preparations

General Procedures. Melting points are uncorrected. NMR spectra were recorded on a General Electric QE-300 spectrometer, operating at 300 MHz. Chemical shifts (δ) are given in ppm using the residual proton signal of the deuterated solvent as reference (CHD₂OD δ 3.300, CHCl₃ δ 7.260, HDO δ 4.80), or the ¹³C signal of the solvent (CD₃OD δ 49.00, CDCl₃ δ 77.00). All solvents were reagent grade quality. Where dry solvents were needed, these were distilled from CaH₂ or Na before use.

Adamantan-1-ylcyanamide was prepared from the amine with cyanogen bromide in Et₂O, as previously described by Geluk, N.W., et al., J. Med. Chem., 1969, 12, 712-716. (2-Methylphenyl)cyanamide was prepared similarly.

N,N'-Di-(adamantan-1-yl)guanidine hydrochloride was prepared according to the procedure of Geluk et al.

### 1. N,N'-Di-(4-bromo-2-methylphenyl)-guanidine (4-Br-DTG)

To a stirred solution of cyanogen bromide (846 mg, 8.06 mmol) in distilled water (70ml) was added in small portions 2.997g (16.11 mmol) of 4-bromo-2-methylaniline (Aldrich, recrystallized from ether-pentane). A white precipitate formed during the addition. The mixture was stirred at 80^{o}C for 4 h. Upon cooling at 0^{o}C for 12 h, a sticky yellow oil separated out and was discarded. The clear aqueous phase was concentrated to about 30 ml. The white precipitate which formed was redissolved by heating the mixture. This was then set aside at 4^{o}C for 12 h. Filtration gave 430 mg of white solid. A 200-mg portion was dissolved in 10 ml of hot water and treated with 5 ml of 10% KOH solution. The mixture was extracted with CHCl₃ and the extract was washed with brine and then dried (MgSO₄). Evaporation of the solvent gave 171 mg of a brown solid which was crystallized from CHCl₃, giving 120 mg (8%) 4-Br-DTG as small white needles: mp 209-210^{o}; NMR (300 MH_{Z}, CD₃OD, TMS) delta 2,24 (s, 3), 7.12-7.25 (AB, 2, J = 8 Hz), 7.35 (s,1); IR (KBr) 3460, 3340, 1630 cm₋¹. Analysis calculated for C₁₅H₁₅N₃Br₂: C, 45.37; H, 3.81; N, 10.58. Found: C, 45.34; H, 3.56; N, 10.50.

### 2. [³H]-N,N'-di-ortho-tolyl-guanidine ([³H] DTG)

Twenty-five mg (0.1 mmol) of the thus-produced 4-Br-DTG were submitted to Amersham Corporation (Arlington Heights, Ill.) for catalytic reduction in the presence of 20 Ci of [³H]-gas. Two mCi portions of the crude, radioactive product in 0.2m/each of 25% ethanol were purified by reverse phase high performance liquid chromatography (RP-HPLC) on a Vydac TP218 octadecasilica column using a CH₃CN gradient (0-35% in 60 minutes) in 0.1% trifluoroacetic acid for elution. Flow rate was 1 ml/min. One minute fractions were collected. Aliquots of the fractions were diluted 100 fold and 10ul aliquots of the diluted fractions corresponding to 0.1ul of the original fractions were dissolved in 10 ml scintillation fluid and counted in a scintillation spectrometer. The HPLC equipment consisted of 2 Waters HPLC pumps; and automated electronic gradient controller and a Kratos variable wave length UV spectrophotometer. The radioactivity eluted as a major, symmetrical peak coinciding with a major, symmetrical UV (220 nm) absorbing peak at 41 minutes. This is the same elution time at which authentic, unlabeled DTG emerges from the column in this RP HPLC system. The specific activity of [³H]DTG was found to be 52 Ci/mmol based on the amount of DTG under the major UV absorbing peak as determined by quantitative UV-spectrophotometry and the amount of radioactivity associated with this peak as determined by quantitative liquid scintillation spectrometry.

### 3. N-(2-Methyl-4-isothiocyanatophenyl-N'-(2-methylphenyl)guanidine [Di-tolyl-Guanidine-Isothiocyanate(DIGIT)]

### a. N-(2-Methyl-4-nitrophenyl)-N'-(2-methylphenyl)guanidine Hydrochloride and the corresponding free base

A vigorously stirred mixture of 2-methylphenyl-cyanamide [1.107 g, 0.380 mmol, prepared from o-toluidine by the method of Safter, et al. (1948)] and 2-methyl-4-nitroaniline hydrochloride in chlorobenzene (45 ml) was heated at 90^{o} for 3 h and then allowed to cool to 25^{o}. The resulting pale yellow precipitate was collected, washed with CH₂Cl₂, and dried, giving 2.284 g (91%) of the hydrochloride of the desired product (pure by NMR). A 681-mg sample was recrystallized twice from absolute EtOH to give 600 mg (88%) of the desired product as pale yellow microcrystals suitable for the next reaction: mp 196-200^{o}; NMR (CD₃OD, 300 MHz) 2.37 (s, 3), 2.47 (S, 3), 7.33-7.40 (m, 4), 7.59 (d, 1), 8.18 (dd, 1), 8.27 (d, 1). A 473-mg sample of the hydrochloride was dissolved in 50 ml of hot water, filtered, cooled to 25^{o}, and treated with 5 ml of 5 N NaOH. The resulting bright yellow precipitate of the tile compound (free base) was dried (403 mg, 92%) and then recrystallized twice from 95% EtOH to give the analytical sample as yellow platelets: mp 177-179^{o}; NMR (DC₃OD, 300 MHz) 2.31 (s, 6), 7.01-7.35 (m, 5), 7.98 (dd, 1), 8.06 (d, 1). Analysis calculated for C₁₅H₁₆N₄O₂: C, 63.37; H, 5.67; N, 19.71. Found: C, 63.41; H, 5.42; N, 19.90.

### b. N-(2-Methyl-4-aminophenyl)-N'-(2-methylphenyl)guanidine hydrochloride

A Parr hydrogenation flask was charged with a solution of N-(2-methyl-4-nitrophenyl)-N'-2-(methylphenyl)-guanidine hydrochloride (478 mg) in 30 ml of absolute EtOH. 30% Pd on charcoal (71 mg) was added and then the mixture was hydrogenated at 60 psi at 25^{o} for 12 h. All further operations were carried out under an atmosphere of Ar. The mixture was centrifuged, filtered through Celite and the filtrate was concentrated in vacuo to 2 ml. Ether (21 ml) was added and after 4 h at 20^{o}, the white precipitate was collected and dried, giving 406 mg (78%) of the title compound (amine hydrochloride) suitable for the next reaction: mp 232.5-234.5^{o}, NMR (CD₃OD, 300 MHz) delta 2.22 (s, 3), 2.34 (s, 3), 6.60 (dd, 1), 6.66 (d, 1), 6.98 (d,1, 7.26-7.38 (m,4).

### c. N-(2-Methyl-4-isothiocyanatophenyl)-N'-(2-methylphenyl)-guanidine Hydrochloride (DIGIT)

NaOAc (1.767 g, 21.5 mmol) and HOAc (0.839 g, 14.0 mmol) were dissolved in dry MeOH such that the final volume of the solution was 100 ml. A 12.7 ml aliquot of this solution was added to 199 mg (0.686 mmol) of N-(2-methyl-4-aminophenyl)-N'-(2-methylphenyl)-guanidine. hydrochloride under an Ar atmosphere. Thiophosgene (54.7 ul, 86.7 mg, 0.754 mmol, freshly distilled) was then injected into the stirred reaction mixture. The reaction was complete upon mixing as judged by silica gel TLC (500:100:1,CHCl₃-MeOH-HOAc). Water (20 ml)was added and the mixture was concentrated in vacuo to 19 ml.

The next steps were done in rapid succession in order to minimize the possible reaction of the isothiocyanate group with the guanidine free base grouping on another molecule. The above 19 ml concentrate was cooled to 0^{o} and treated with ice-cold saturated NaHCO₃(15 ml). The resulting mixture containing a white precipitate was extracted with CHCl₃ (4 x 15 ml). The combined extracts were washed with ice cold brine, dried (MgSO₄), filtered through Celite, and cooled to 0^{o}. Next excess HCl gas was bubbled thorugh the colorless solution and then the solution was concentrated in vacuo to 25 ml and hexane (20 ml) was added. Four additional times the mixture was concentrated again and more hexane was added. Evaporation of the final mixture to dryness gave crude hydrochloride 5 as a gummy white solid (263 mg, 115%). This was taken up in absolute ethanol (2 ml), and diluted with ether (80 ml) to give a cloudy white suspension from which small clusters of white needles formed on standing. The crystals were centrifuged, washed with ether (3 x 5 ml), and dried, giving 173 mg (76%) of the title compound: m.p. 195-197^{o}C (preheated bath); NMR (CD₃OD), 300 MHz) delta 2.347 (s, 3), 2.351 (s, 3), 7.23 (d, 1), 7.31 (s, 1), 7.34 (d, 1), 7.26-7.40 (m, 4); IR (KBr) 3466, 3138, 2927, 2152, 2119, 1646, 1631 cm⁻¹. Analysis calculated for C₁₆H₁₇ClN₄S: C, 57.74; H, 5.15; N, 16.83. Found: C, 57.60; H, 5.20; N, 16.64.

A tri-tritiated version of DIGIT is Prepared starting with tritiated N-(2-methyl-4-aminophenyl)-N'-(2-methylphenyl)-guanidine, which was prepared by catalytic tritiation (Amersham) of N-(2-methyl-4-nitro-6-bromophenyl)-N'-(2-methyl-4,6-dibromophenyl)-guanidine. The tri-tritiated amino compound was also used as the immediate precursor for the preparation of the tri-tritiated version of the 4-azido compound whose preparation is described hereinafter and which was used in the solubilization of sigma receptors.

### N-Adamantan-1-yl-N'-cyclohexylguanidine HCl

Adamantan-1-ylcyanamide (514 mg, 2.92 mmol), and cyclohexylamine hydrochloride (398 mg, 2.93 mmol) were finely ground together, and heated at 200 ^{o}C for 10 min. The resultant glassy solid was pulverized, and extracted twice with 50 mL boiling 5% HCl. The insoluble material was filtered, and dried. 346 mg, mp 264-270^{o} (p.h.b. 240^{o}). On cooling the combined aqueous extracts to 25^{o}, a white ppt. formed, and was filtered off, 56 mg. Combined crude yield: 44%. Crystallization of a 50 mg sample of the crude product from EtOH/Et₂O afforded white needles (25 mg, mp 269-271 ^{o}C (p.h.b. 240^{o}), lit. 268-269^{o}C). ¹H NMR (CD₃OD) 1.208-1.474 (m, 5H0, 1.744 (s, 9H), 1.967 (s, 8H), 2.119 (s, 3H), 3,434 (m, 1H0, ¹³C NMR (CD₃OD) (broad band decoupled) 25.55, 26.26, 30.96, 33.74, 36.74, 42.73.

### N-Cyclohexyl-N'-(2-methylphenyl)guanidine

A suspension of cyclohexyl amine hydrochloride (310 mg, 2.28 mmol) and (2-methylphenyl)cyanamide (365 mg, 2.76 mmol) in dry chlorobenzene was heated at 125-135^{o} for 4 hrs. The solvent was evaporated in vacuo with heating, and the residue partitioned between CH₂Cl₂ and 20 x 8 ml 10% HCl. The aqueous extracts were made alkaline to pH 9-9.5, adn the white precipitate collected after standing at 4^{o}C overnight. Two recrystallizations from EtOH/H₂O gave 140 mg (22%) of white needles, mp 145-146^{o}, ¹H NMR (CD₃OD) 1.149-2.054 (m, 10 H), 2.164 (s, 3 H), 3.50 (M; 1 H), 6.875 (d, 1H, J = 7.5 Hz), 6.968 (t, 1H, J = 7.5 Hz), 7.118 (t, 1H, J = 7.5 Hz), 7.177 (d, 1H, J = 7.5 Hz). Anal. Calcd for C₁₄H₂₁N₃: C, 72.69; H, 9.15; N, 18.16. Found: C, 72.72; H, 9,24; N, 18.18.

### N-(Adamantan-1-yl)-N'-(2-methylphenyl)guanidine.

A suspension of adamantan-1-ylcyanamide (151 mg, 0.857 mmol) and o-toluidine hydrochloride (186 mg, 0.857 mmol) in dry chlorobenzene was heated between 100-130^{o}C for 4.5 hrs. The chlorobenzene was evaporated in vacuo with heating and the residue (285 mg) taken up in 18 ml of water. A gummy, insoluble material was discarded. On adjusting the aqueous extract to pH 9-9.5, a precipitate formed (194 mg, 71%). Two recrystallizations from EtOH/H₂O gave the analytical sample, mp 160-161^{o}, ¹H (CD₃OD) 1.742 (s, 6 H0, 2.050 (d, 6 H, J = 2.4 Hz), 2.029 (s, 3 H0, 6.956 (j, 1 H, J =- 8.1 Hz), 7.056 (t, 1H, J - 3.1 Hz), 7.168 (t, 1H, J = 7.a5 Hz), 7.213 (d, 1H, J = 7.5 Hz). Anal. Calcd for C₁₈H₂₅N₃: C, 76.28; H, 8.89; N, 14,83. Found: C, 76.25; H, 8.86; N, 14.60.

### N-(adamantan-1-yl)-N'-(2-iodophenyl)guanidine HCl

Adamantan-1-ylcyanamide (299 mg, 1.70 mmol) and o-iodoaniline hydrochloride (433 mg 1.70 mmol) were finely ground together and heated at 200^{o} for 10 min. The resultant black glassy solid was pulverized, and recrystallized 5 times from EtOH/Et₂O. The resultant faintly blue needles (80 mg) were dissolved in 4 ml EtOH, and passed through a short (< 1 cm) column packed from bottom to top with Celite, charcoal, activated alumina, and sand. The colorless eluate was then diluted with 5 ml Et₂O, and allowed to stand in an Et₂O diffusion chamber overnight. The white needles were collected by suction filtration, 40 mg, mp 274-275^{o}C. Addition of another 5 ml Et₂O yielded a second crop (31 mg) of identical material. Combined yield: 21%. ¹H NMR (CO₃OD) 1.779 (s, 6 H), 2.090 (s, 6H), 2.165 (s, 3 H), 7.168 (t, 1H, J = 8.1 Hz), 7.388 (d, 1 H, J = 9.1 Hz), 7.503 (t, 1 H, J = 7.8 Hz), 8.004 (d, 1 H, J = 7.8 Hz).
N-(2-Methyl-4-azido-phenyl)-N'-(2-methylphenyl)guanidine N-(2-Methylphenyl)-N'-(2-methyl-4-amino-phenyl)guanidine dihydrochloride (112 mg, 0.341 mmol) were dissolved in 2 ml H₂O and 100 µl conc. HCl (1.2 mmol). The solution was cooled in an ice bath, and a solution of NaNO₂)42 mg, 0.61 mmol) in 450 µl H₂O were added. The reaction mixture turned yellow, and was stirred for 45 min before solid NaN₃ (43 mg, 0.661 mmol) was added in a single portion. After N₂ evolution had ceased, a foamy solid (11 mg) was removed and discarded. Solid NaOH (96 mg, 2.41 mmol) was added, and the bright yellow precipitate was extracted with Et₂O (3x5 ml). Evaporation of the combined Et₂O layers gave a yellow solid which was crystallized from EtOH/H₂O. NMR (CD₃OD) 2.279 (s, 3 H), 2.284 (s, 3 H), 6.864 (dd, 1 H, J = 2.4 Hz, 8.4 Hz), 6.914 (d, 1 H, J - 2.1 Hz), 7.055 (td, 1 H, J = 1.8 Hz, 7.2 Hz), 7.136-7.229 (m, 4 H).

### N-(2-Methyl-4-nitro-6-bromophenyl)-N'-(2-methyl-4,6-dibromophenyl)guanidine.

N-(2-methyl-4-nitrophenyl)-N'-(2-methylphenyl)guanidine, as the free base, ² 281 mg, 0.987 mmol) was dissolved in 4 ml MeOH, and cooled in an ice-bath. N-bromosuccinimide (freshly recrystallized from H₂O) (531 mg, 2.98 mmol) was added in two portions over 15 min. After 1.5 hrs the brown sludgy reaction mixture was diluted with 4 ml MeOH, and allowed to warm to 25 ^{o}C. A brown solid was filtered off (266 mg), and crystallized from acetone/H₂O, to afford brown needles (2.6 mg, 42%, mp 193-195^{o}C). Sublimation of a 56 mg sample of these crystals at .01 mm Hg and 170^{o} afforded the analytical sample as a bright yellow amophrous solid (38 mg, mp 210-213^{o}C). NMR (CD₃OD) 2.357 (s, 3 H), 2.488 (s, 3 H), 7.444 (d, 1 H, J- 1.5 Hz), 7.669 (d, 1H, J = 1.8 Hz), 8.033 (d, 1H, 2.1 Hz), 8.267 (d, 1H, 2.4 Hz). Anal. Calcd for C₁₅H₁₃Br₃N₄O₂: C, 34.58; H, 2.52; N, 10.75. Found: C, 34.64; H, 2.47; N, 10.65.

### Characteristics of [³H]DTG binding to guinea pig brain membranes

Synthesis of [³H]DTG resulted in a pure homogenous product of high specific radioactivity (52 Ci/mmol). [³H]DTG bound specifically, saturably, reversibly, and with high affinity to guinea pig brain membrane. In a typical experiment with 0.9 nM [³H]DTG ( 30,000 cpm, 50% counting efficiency) the total binding was 2,700 cpm while the nonspecific binding in the presence of 10 uM DTG or 10 uM haloperidol was 50-150 cpm. Routinely, a specific binding to 90-97% of total binding was observed. At room temperature the binding of [³H]DTG reached equilibrium after 60-90 minutes and it was fully reversible after addition of 10 uM unlabeled DTG. Specific binding was linear with tissue concentration between 2-40 mg tissue (original wet brain weight per assay tube). Binding of radioactivity to the glass fiber filters in the absence of membranes was 10-20 cpm. Boiling of membranes at 100^{o}C for 10 minutes prior to assay almost completely ( 90%) abolished specific [³H]DTG binding as did treatment of the membranes with trypsin and pronase (0.01 mg/ml for 30 min at room temperature), indicating that protein components are important for the receptors' binding ability.

To determine the equilibrium saturation binding of [³H]DTG to guinea pig brain membranes, membranes prepared as described herein were incubated with [³H]DTG at various concentrations from 0.3 mM to 90 mM in 1 ml 50 mM Tris/HCl buffer, pH 7.4, for 120 minutes at room temperature. Values obtained were the mean of quadruplicate determinations.

A Scatchard analysis of the saturation data shows a linear Scatchard plot with an apparent K_{D} of 28 mM and a maximum number of binding sites (Bmax) of 84 pmol/g brain tissue (original wet weight). Analysis of the binding data with the curve fitting program LIGAND, Munson, P.J. & Rodbard, D., Anal. Biochem. 107, 220-239 showed high compatibility with a one site binding model.

### Radioligand Binding Assays

Frozen guinea pig brains (Pel-Freeze, Rogers, AK) were homogenized in 10 volumes (w/v) of 0.32M sucrose using a Polytron homogeniser. The homogenate was spun at 900 x g for 10 minutes at 4^{o}C. The supernatant was collected, and spun at 22,000 x g for 20 minutes at 4^{o}C. The pellet was resuspended in 10 volumes of 50 mM Tris/HCl buffer, pH 7.4, incubated at 37^{o}C for 30 minutes and spun again at 22,000 x g for 20 minutes at 4^{o}C. The pellet was then resuspended in 10 volumes of 50 mM Tris/HCl buffer, pH 7.4 and 10 ml aliquots of this membrane suspension were stored frozen at -70^{o}C until used in the binding assay. No effects of prolonged storage (> 3 months) of the membranes at -70^{o}C on sigma receptor number or affinity for [³H]DTG binding were observed.

For radioreceptor assays aliquots of the frozen membrane suspension were thawed and diluted tenfold with 50 mM Tris/HCl buffer, pH 7.4. To 12 x 75 mm polystyrene or glass test tubes were added 0.8ml of membrane suspension, 0.1 ml [³H] DTG or (+)[³H]3-PPP for a final concentration of 0.9 mM, and 0.1 ml of unlabeled drugs or buffer. The protein concentration in the 1 ml final incubation volume was 800 ug, corresponding to 32 mg of brain tissue (original wet weight). Nonspecific binding was defined as that remaining in the presence of either 10 uM DTG or haloperidol for both the [³H]DTG and the (+)[³H]3-PPP binding. After incubation for 90 minutes at room temperature the membrane suspensions was rapidly filtered under vacuum through Whatman GF/B glass fiber filters using a Brandel 48 well cell harvestor (Brandel, Gaithersburg MD). The filters were washed with 3 x 5 ml ice-cold 50 mM Tris buffer (pH 7.4 at room temperature). The filters were dissolved in 10 ml each of Cytoscint (Westchem Products, San Diego, CA) and radioactivity was measured by liquid scintillation spectrometry at a counting efficiency of 35-50%. Saturation data were evaluated by Scatchard analysis using both the EBDA McPherson, G.A., (1983) Computer Programs Biomed. 17, 107-114 and LIGAND Munson, P.J. & Rodbard, D., (1980) Anal. Biochem. 107, 220-239, data analysis programs on an IBM Personal Computer-AT. IC50 values were determined by plotting displacement curves onto semilogarithmic graph paper followed by interpolation.

### Drug Specificity of [³H]DTG Binding

Displacement experiments were performed with drugs that are considered typical sigma ligands, as well as with drugs considered to be prototypical ligands for other neurotransmitter, neuromodulator, and drug receptors. These experiments showed that the [³H]DTG binding site was stereoselective for dextrorotatory benzomorphan opiates and for (-)butaclamol; does not significantly interact with drugs that have high affinities for acetylcholine, benzodiazepine, GABA, nor with mu, delta, or kappa opioid receptors; and has a high affinity for haloperidol and several drugs belonging to the phenothiazine class of antipsychotics (haloperidol had the highest displacement potency of all drugs tested); and has a moderate affinity for several other classes of psychoactive drugs, which included several tricyclic antidepressants, PCP, and the kappa-opioid receptor ligand U50, 488H.

**TABLE I**

| Drug | IC₅₀ against [³H]DTG (nM) (±SEM) | IC₅₀ against (+)[³H]3-PPP(nM) (±SEM) |
|---|---|---|
| Haloperidol | 5±0.3 | 17±1 |
| DTG | 28±1 | 53±9 |
| Perphenazine | 42±10 | 21±3 |
| (+) Pentazocine | 43±2 | 8±3 |
| (-) Pentazocine | 135±3 | 81±1 |
| (±) Pentazocine | 69±1 | ND |
| (+) 3-PPP | 76±4 | 33±12 |
| (-) 3-PPP | 280±21 | 235±60 |
| (+) Cyclazocine | 365±25 | 47±12 |
| (-) Cyclazocine | 2,600±210 | 1,000±0 |
| Spiperone | 690±21 | ND |
| (-) Butaclamol | 530±49 | 183±5 |
| (+) Butaclamol | 2,150±250 | 2,100±71 |
| (+) SKF 10,047 | 625±88 | 93±5 |
| (-) SKF 10,047 | 4,000±566 | 2,850±390 |
| PCP | 1,050±106 | 1,000± 71 |
| U50,488H | 1,350±106 | ND |
| Trifluoperazine | 345±4 | ND |
| Triflupromazine | 605±67 | ND |
| Chlorpromazine | 1,475±265 | ND |
| Amitriptyline | 300±7 | ND |
| Imipramine | 520±14 | ND |
| Desipramine | 4,000±212 | ND |
| Nortriptyline | 2,000±640 | ND |
| Guanabenz | 4,600±283 | ND |
| Clonidine | >10,000 | ND |
| Cocaine | >10,000 | ND |

| | | |
|---|---|---|
| *IC₅₀ is the molar concentration of the drug needed to produce half-maximal displacement of [³H]DTG from sigma receptors. This is a direct measure of the sigma receptor binding potency of the drug. ND = not determined. | | |

The above IC₅₀ₛ represent the average from 2-4 separate experiments (in triplicate). The following compounds caused no significant displacement at a 10uM concentration: scopolamine, 5-OH-tryptamine, diazepam, bicuculline, picrotoxin, hexamethonium, dopamine, apomorphine, GABA, gamma-guanidino butyric acid, morphine, DAGO, metorphamide, dynorphin A, [leu⁵] enkephalin, beta-endorphin, naloxone, guanidino acetic acid, creatine, creatinine, 1,1-di-methyl-guanidine, methyl-guanidine, beta-guanidino propionic acid and cimetidine.

### Drug specificity of [³H]DTG binding compared to (+)-[³H]3-PPP binding.

Comparing the drug specificity of [³H]-DTG binding with that of (+)[³H]3-PPP in the guinea pig, it was found that (+)[³H]3-PPP bound specifically, saturably (linear Scatchard plot), reversibly and with high affinity to guinea pig brain membranes (K_{D}=30nM; Bmax = 80 pmol/g fresh brain weight) The drug specificity profile of the (+)[³H]3-PPP binding in the guinea pig (TABLE I) was found to be very similar to that reported in the rat. Largent et al., (1984) supra. Moreover, the drug specificity profiles of typical sigma receptor active drugs in the (+) [³H]3-PPP and [³H]-DTG binding assays were highly correlated (r=0.95;p≦0.00001) which is consistent with the two compounds labeling the same sites.

### Autoradiography Studies

Male Sprague Dawley rats (200-250g) and NIH guinea pigs (300-350g) were sacrificed, their brains rapidly removed and processed for receptor autoradiography according to the method of Herkenham and Pert, Herkenham, M. & Pert, C.B., (1982) J. Neuroscience 2, 1129-1149.

Fifteen µm thick slide-mounted brain sections were incubated for 45 minutes in 50 mM Tris-HCl (pH 8.0, 22^{o}C) containing 1 mg/ml bovine serum albumin (BSA) and 2 nM [³H]DTG. Adjacent sections were incubated with 10 uM haloperidol or 10 uM DTG to measure nonspecific binding. Incubations were terminated by 4x2 minute washes in 10 mM Tris-HCL (pH 7.4, 4^{o}C) with 1mg/ml BSA, rapidly dried under a stream of cool air and placed in x-ray cassettes with ³H-sensitive film (³H-Ultrofilm, LKB). Films were developed 6-8 weeks later (D-19, Kodak).

### Autoradiographic visualization of [³H]DTG binding.

Receptor autoradiography studies on guinea pig and rat brain sections using [³H]DTG showed a low density of specific binding diffusely distributed throughout the gray matter of the rat and ginea pig brain. Superimposed on this homogeneous binding patters was a heterogeneous distribution of enriched binding in limbic and sensorimotor structures. The pattern of binding was more distinct in the guinea pig than rat. Similar observations for (+) [³H]3-PPP autoradiography have been reported. Largent et al., (1986) supra. Thus, description of [³H]DTG binding was drawn primarily from the guinea pig. In the forebrain, limbic structures moderately to densely labeled by [³H]DTG were the diagonal band of Broca, septum, hypothalamus (especially the paraventricular nucleus), anterodorsal thalamic nucleus and zona incerta. Sensorimotor thalamic nuclei moderately to densely labeled included the thalamic taste relay and reticular nuclei. Other thalamic nuclei labeled were the paraventricular and habenular nuclei. Very dense binding was seen in the choroid plexus. In the cortex dense [³H]DTG labeling occupied layer III/IV of retrosplenial piriform, and entorhinal cortices. The rest of the cortex contained a low level of homogeneous binding. The hippocampal formation exhibited discrete binding in the pyramidal granular cell layers. Sensorimotor areas of the midbrain were selectively labeled by [³H]DTG. The oculomotor nucleus, and more caudally, the trochlear nucleus were very densely labeled, and the superior colliculus and red nucleus had moderate levels of binding. Other midbrain nuclei labeled were the dorsal raphe, interpeduncular nucleus, central gray, and the substantia nigra, spars compacta. The selective labeling of the pars compacta in the guinea pig contrasted with the low to moderate density of labeling present throughout the substantia nigra of the rat. In addition, very dense binding was found in the subcommissural organ. In the hindbrain the locus coeruleus was the most densely labeled nucleus. Sensorimotor nuclei enriched in [³H]DTG binding sites were the trigeminal motor nucleus, nucleus of the facial nerve, nucleus of the solitary tract, dorsal motor nucleus of the vagus, and hypoglossal nucleus. Moderate to dense binding was also found throughout the gray matter of the cerebellum, and in the pontine reticular nuclei.

### Drug specificity of [³H]AZ-DTG binding

The haloperidol-sensitive sigma receptor binds [³H](+)3-[3-hydroxyphenyl]-N-(1-propyl)piperidine([³H](+)-3 -PPP) and [³H]1,3-di-o-tolylguanidine ([³H]DTG), with high affinity. In order to elucidate its structure, photoaffinity labeling of the sigma receptor from guinea pig brain was accomplished using a novel radioactive photolabile derivative of DTG, [³H]-m-azido-1,3-di-o-tolylguanidine ([³H]AZ-DTG). In the dark, [³H]AZ-DTG binds reversibly to sigma sites in brain membranes with high affinity (K=28 nM). The drug specificty profile of [³H]AZ-DTG binding to brain membranes is identical to that of the prototypical sigma ligands [³H]DTG and [³H](+)-3-PPP. For photoaffinity labeling, membrane suspensions containing protease inhibitors were preincubated in the dark with [³H]AZ-DTG, then filtered and washed over Whatman GF/B glass fiber filters. The filters were then irradiated with long-wavelength Uv light for a 15 minute period. Filter-bound proteins were solubilized with 50 mM Tris pH 7.4, 0.1% sodium dodecyl sulfate. Solubilized proteins were subjected to SDS-polyacrylamide gel electrophoresis. Fluorography of the SDS-PAGE gels revealed that [³H]AZ-DTG was selectively incorporated into a 29 kD polypeptide. Labeling of this polypeptide was completely blocked by the sigma ligands DTG, (+)-3-ppp, (+)pentazocine, and haloperidol at a concentration of 10 uM, while labeling was unaffected by morphine, serotonin, dopamine, scopolamine, or GABA at the same concentration. These results represent the first estimate of the size of the binding subunit of the haloperidol-sensitive sigma receptor.

### Augmentation of electrically evoked norepinephrine release in the mouse vas deferns

Male Swiss Webster mice weighing 25-30 g were killed by decapitation; their vasa deferentia were dissected out, cleaned and mounted in 5 ml organ baths at a resting tension of 250 mg. The tissues were bathed with modified Krebs solution [composition (mM): NaCl 118, KCl 4.75, CaCl₂ 2.54, NaHCO₃ 25.0, KH₂PO₄ 0.93, D-glucose 11.0, and naloxone HCl 0.001] maintained at 37^{o}C and bubbled with 95% O₂ and 5% CO₂. Tissues were stimulated by a Grass S88 stimulator at 0.1 Hz (supramaximal voltage, 1 msec duration) via platinum ring electrodes 2 cm apart, and contractions were recorded isometrically via Grass FT .03 transducers attached to a Grass polygraph. Agonist potency of drugs was determined by application of single doses and measurement of twitch height when the response to drug had stabilized, followed by drug washout by overflow. For studies in which NE concentration was determined, the above protocol was followed with these modificatoins: six vasa were mounted in parallel in one 5 ml bath at a resting tension of 1.0 g and tissues remained in the presence of the agonist dose for 20 minutes, at the end of which the bathing fluid was collected, rapidly frozen on dry ice and subjected subsequently to high performance liquid chromatography with electrochemlcal detection for analysis of NE content.

(+)3-ppp, TCP when applied to the MVD bathing solution cause an augmentation of the electrically evoke twitch amplitude that is rapid in onset, reach a stable plateau in 2-5 minutes and is rapidly reversed by washing the compound away. Responses to (+)3-PPP increase in a dose-dependent manner to a stable, reproducible maximum of 251.0 + 3.1 uM SEM (n=15). Responses to TCP increase to a maximum of 338.2 + 27.4 SEM (n=12) with an EC₅₀ of 27.6 + 3.2 uM SEM (n=12). Measurement of NE content in the bathing solution following 20 minute incubation in the presence of (+)3-PPP reveals significant increases at 200 uM. Ne content following incubation of 200 uM TCP also increases significantly.

### Functional Sigma Receptors on Noradrenergic Nerves Terminals of the Mouse Vas Deferens

(+)3-PPP and TCP,act in the mouse vas deferens (MVD) to augment the electrically evoked release of NE. The receptor specificity of these ligands demonstrated at central binding sites suggests they act in the MVD at distinct receptors to produce a common final response. One mechanism proposed by Bartschat and Blaustein, Proc. Nat. Acad. Sci. U.S.A., 1966, 83:189-192), for PCP action consistent with these findings is inhibition of voltage-regulated, non-inactivating K+ channels, resulting in action potential prolongation and augmentation of neurotransmitter release. The mechanism of action of sigma receptors has not previously been characterized. We have found that MVD provides a useful model system to identify competitive antagonists at this site.

The sigma receptor binding site can be selectively labelled with (+)[³H]3-hydroxyphenyl-N(1-propyl)-piperidine ((+)[³H]3-PPP) and with tritium, labelled N,N'-disubstituted guanidines which possess sigma receptor binding activity, e.g., [³H]1,3-di-o-tolyl-guanidine ([³H]DTG). The physiological function of this binding site was heretofore unknown. We have found that the (+)3-PPP augmentation of the electrically stimulated release of [³H)norepinephrine [³H]NE from preloaded mouse vas deferens (MVD) is competitively antagonized by N,N'-disubstituted guanidines which are antagonists to hallucinogenic sigmabenzomorphanes, e.g., by DTG, with a K_{E} of 29nM, and by N,N'-di-o-phenyl-guanidine (DPG), with a K_{E} of 400nM. This compares to a K_{D} for DTG of 28nM and a Kᵢ for DTPG of 400n in brain membrane binding assays. This suggests that in the MVD there are functionally active sigma receptors which mediate an augmentation of electrically stimulated norepinephrine (NE) release, and that DTG and DPG are competitive antagonists at sigma receptors. The sigma receptor bioassay system and the N,N'-disubstituted guanidine antagonists of this invention are useful tools to determine the actions of benzomorphans and other drugs on sigma receptors and to investigate the physiological role of these receptors.

As described above, the selective sigma ligand (+)3-PPP causes a dose-dependant increase in electrically stimulated NE release in the MVD as determined by subjecting the MVD organ bath fluid to NE analysis using reversed phase high performance liquid chromatography with electrochemical detection (RP-HPLC/EC)¹³. In order to study this presynaptic action of (+)3-PPP in more detail we preloaded the NE nerve terminals of the MVD by incubating the vasa with [³H]NE (25uCi/ml/4 vasa) in Krebs/Ringer buffer containing 30uM desoxycorticosterone acetate (to block uptake of [³H]NE into the smooth muscle tissue) at 37^{C} for 30 min with continuous bubbling of 95% O₂/5%CO₂. Four vasa per experiment were then mounted individually in 5ml organ baths in 95% O₂/5% CO₂ saturated Kreba/Ringer buffer and maintained at 37^{o}C.

(+)3-PPP causes a dose-dependent augmentation of electrically stimulated release of [³H]NE into the bath fluid. When 10uM DTG, a selective sigma receptor ligand is added to the bath fluid together with varying doses of (+)3-PPP, the effect of (+)3-PPP on the release of [³H]NE is completely abolished, which confirms that DTG is an antagonist of (+)3-PPP's effect on the release of [³H]NE associated radioactivity in the MVD. In brain membrane binding assays, (+)[³H]3-PPP and [³H]DTG both selectively label the same binding site. This binding site fulfills the pharmacological criteria of sigma receptors in that it displays affinity for benzomorphan opiates known to have sigma-like behavioral effects but not for morphine and other classical opiates which are devoid of sigma-like behavioral effects. Furthermore, DTG competitively displaces [³H](+)3-PPP, and (+)3-PPP competitively displaces [³H]DTG from sigma binding sites. Because (+)3-PPP's effect on [³H]NE release in the MVD is mediated by sigma receptors and because DTG is an antagonist, as evidenced by the fact that DTG abolishes (+)3-PPP's action, then DTG's antagonism of (+)3-PPP is competitive, as evidenced by the dose/response curve of (+)3-PPP's effect on [³H]NE release in the presence of 120nM DTG, which results in a five-fold shift to the right compared to the (+)3-PPP control dose/response curve in the absence of DTG. A Schild analysis of the (+)3-PPP dose/response relationship in the absence of DTG and in the presence of 70, 270 and 960 nM DTG results in appropriately parallel shifts of the (+)3-PPP dose/response curves to the right. The pA₂ value for the DTG antagonism derived from the Schild plot corresponds to an antagonist affinity (K_{E}) of 29nM. The Schild slope was 0.85.

1,3-Di-phenyl-guanidine (DGPG) was for antagonism of (+)-PPP's effect on [³H]NE release. DPG also binds to sigma receptors in binding assays but has a lower affinity than DTG. 80uM DPG results in a complete block of (+)3-PPP's effect on [³H]NE release, while 1.6 uM DPG results in a five-fold shift of the (+)3-PPP dose/response curve to the right. A Schild analysis at 920, 3,600 and 12,800 nM gave a pA₂ value corresponding to a K_{E} of 400 nM. A number of control experiments ruled out (+)3-PPP's action being attributable to any known mechanisms which cause an increase in NE release such NE uptake inhibition or alpha-2 adrenergic receptor blockade.

The postulation that a functionally active receptor the potency (K_{E}) of a competitive antagonist in an appropriate bioassay system should correspond to its affinity (K_{d}) in an appropraite binding assay whereas the potency of an agonist is usually lower in the bioassay compared to its affinity in a binding assay, postulate is fulfilled in the bioassay system described here. The K_{E} values for the two competitive antagonists DTG and DPG correspond almost perfectly to their K_{D}/Kᵢ values in a sigma receptor binding assays using [³H](+)3-PPP or [³H]-DTG to label the receptors in brain membrane suspensions. In contrast, the EC₅₀ for (+)3-PPP's agonist activity is three orders of magnitude lower than its binding affinity. Taken together, these findings establish that the [³H]NE releasing action of (+)3-PPP is a true receptor mediated effect and that the receptors which mediate this action are likely to be similar or identicla to the sigma receptors which are selectively labelled in brain membrane suspensions by [³H](+)3-PPP and [³H]DTG.

The preceding examples can be repeated with similar success by substituting the generically or specifically described reactants and/or operating conditions of this invention for those used in the preceding examples.

## Claims

1. Use of an N,N'-disubstituted guanidine derivative of the formula I 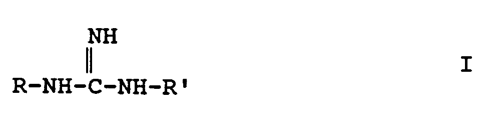 wherein R and R', which can be the same or different, are alkyl of 4 to 12 carbon atoms, cycloalkyl of 3 to 12 carbon atoms, carbocyclic aryl, alkaryl or aralkyl of up to 18 carbon atoms, whereby the R and R' hydrocarbon groups may bear 1, 2 or 3 of the following substituents: alkyl of 1 to 8 carbon atoms, chloro, bromo, iodo, fluoro, nitro, azido, cyano, isocyanato, amino, lower-alkylamino, di-lower-alkylamino, trifluoromethyl, alkoxy of 1 to 8 carbon atoms, acyloxy of 1 to 8 carbon atoms, amido, N-ethylacetamido, carbamido, N-methylcarbamyl, and N,N'-dimethylcarbamyl, or N-(2-Methyl-4 isothiocyanatophenyl)-N'-(2-methylphenyl)-guanidine for the preparation of a pharmaceutical composition useful for the diagnosis and treatment of psychosis.

2. An N,N'-disubstituted guanidine derivative of the formula I as described in claim 1 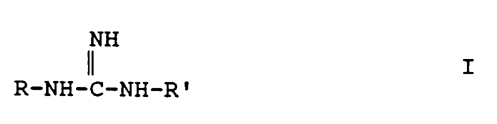 wherein R and R' are each an alkyl group of at least 4 carbon atoms, adamantyl, cyclohexyl or a carbocyclic aryl of at least 6 carbon atoms and wherein at least one of the ring carbon atoms of R and R' bears at least one tritium atom.

3. The compound of claim 2, which is N,N'-di-(4-[³H]-2-methylphenyl)-guanidine.

4. Use according to claim 1, wherein, in the guanidine derivative used, R and R' are each an alkyl group of at least 4 carbon atoms, a cycloalkyl group of 3 to 12 carbon atoms or a carbocyclic aryl group of at least 6 carbon atoms.

5. A method for determining the sigma brain receptor binding activity of an organic compound which comprises the steps of:
a) contacting in an aqueous medium a known amount of isolated mammalian brain membrane which has psychotomimetic benzomorphan binding activity, with a mixture of (i) a tritium-labeled N,N'-disubstituted guanidine according to claim 2 which selectively binds sigma brain receptors, in a known amount capable of being bound to the sigma receptors of that brain membrane; and (ii) varying known amounts of a water-soluble organic compound to be assayed for sigma receptor binding activity;
b) separating the brain membrane from any of the tritium-labeled compound which is not bound to the brain membrane in step a);
c) determining, from the molar relationship of the proportion of bound tritium-labeled compound which is separated in step b) to the molar amount of the organic compound employed in step a), the sigma receptor binding activity of that organic compound.

6. A method according to claim 5, wherein the tritium-labeled compound is a disubstituted-guanidine of the formula 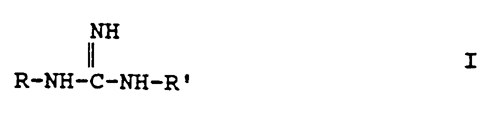 wherein R and R' are each an alkyl group of at least 4 carbon atoms or carbocyclic aryl groups of at least 6 carbon atoms.

7. A method according to claim 5, wherein the tritium-labeled compound is N,N'-di-(4-[³H]-2-methylphenyl-guanidine.

8. Use according to claim 1 or 4 wherein the pharmaceutical composition prepared is useful in determining the relationship of abnormal psychotic-like behavior, in a mammal displaying such behavior, to sigma receptor system dysfunction.

9. Use according to claim 1 or 4 wherein the pharmaceutical composition prepared is useful in treating a human being suffering from a psychotic mental illness associated with hallucinations.

10. Use according to claim 9, wherein the disubstituted guanidine used is a compound of the formula 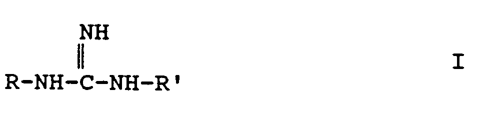 wherein R and R' are each the same and each is an alkyl group of at least 4 carbon atoms, a cycloalkyl group of 3 to 12 atoms or a carbocyclic aryl group of at least 6 carbon atoms.

11. A compound as defined in formula I of claim 1, namely N-cyclohexyl-N'-(2-methylphenyl)-guanidine.

12. A compound as defined in formula I of claim 1, namely N-(adamantan-1-yl)-N'-2iodophenyl-guanidine.

13. A compound as defined in formula I of claim 1, namely N-(adamantan-1-yl)-N'-(2-methylphenyl)-guanidine.

14. A pharmaceutical composition comprising the compound of claim 11 and a pharmaceutically acceptable carrier.

15. A pharmaceutical composition comprising the compound of claim 12 and a pharmaceutically acceptable carrier.

16. A pharmaceutical composition comprising the compound of claim 13 and a pharmaceutically acceptable carrier.

17. Use according to claim 9, wherein said N,N'-disubstituted guanidine is N-cyclohexyl-N'-(2-methylphenyl)-guanidine.

18. Use according to claim 9, wherein said N,N'-disubstituted guanidine is N-(adamantan-1-yl)-N'-iodophenyl-guanidine.

19. Use according to claim 9, wherein said N,N'-disubstituted guanidine is N-(adamantan-1-yl)-N'-(2-methylphenyl)-guanidine.

20. Use according to claim 9, wherein said N,N'-disubstituted guanidine is N-(adamantan-1-yl)-N'-cyclohexyl-guanidine.

21. Use according to claim 4, wherein R is cyclohexyl and R' is o-tolyl.

22. Use according to claim 4, wherein R is adamantanyl and R' is cyclohexyl.

23. Use according to claim 4, wherein both R and R' are adamantanyl.

24. Use according to claim 4, wherein R is adamantanyl and R' is 2-iodophenyl.

25. Use according to claim 4, wherein R is adamantanyl and R' is o-tolyl.

## Patentansprüche

1. Verwendung eines N,N'-disubstituierten Guanidinderivats der Formel I 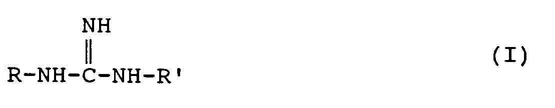 in der R und R', die gleich oder verschieden sein können, Alkylreste mit 4 bis 12 Kohlenstoffatomen, Cycloalkylreste mit 3 bis 12 Kohlenstoffatomen, carbocyclische Arylreste, Alkarylreste oder Aralkylreste mit bis zu 18 Kohlenstoffatomen bedeuten, wobei die Kohlenwasserstoffreste R und R' 1, 2 oder 3 der folgenden Substituenten tragen können: Alkylreste mit 1 bis 8 Kohlenstoffatomen, Chlor-, Brom-, Jod-, Fluoratome, Nitrogruppen, Azidogruppen, Cyanogruppen, Isocyanatgruppen, Aminogruppen, Niederalkylaminoreste, Di-Niederalkylaminoreste, Trifluormethylgruppen, Alkoxyreste mit 1 bis 8 Kohlenstoffatomen, Acyloxyreste mit 1 bis 8 Kohlenstoffatomen, Amidogruppen, N-Ethylacetamidogruppen, Carbamidogruppen, N-Methylcarbamylgruppen und N,N'-Dimethylcarbamylgruppen, oder von N-(2-Methyl-4-isothiocyanatphenyl)-N'-(2-methylphenyl)-guanidin für die Herstellung eines Arzneimittels, das für die Diagnose und Behandlung einer Psychose wertvoll ist.

2. N,N'-disubstituiertes Guanidinderivat der Formel I gemäß Anspruch 1 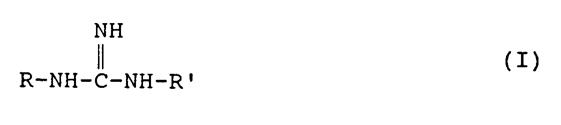 in der R und R' jeweils einen Alkylrest mit mindestens 4 Kohlenstoffatomen, eine Adamantylgruppe, Cyclohexylgruppe oder einen carbocyclischen Arylrest mit mindestens 6 Kohlenstoffatomen bedeuten und wobei mindestens einer der Ringkohlenstoffatome von R und R' mindestens ein Tritiumatom trägt.

3. Verbindung nach Anspruch 2, nämlich N,N'-Di-(4-[³H]-2-methylphenyl)-guanidin.

4. Verwendung nach Anspruch 1, wobei in dem verwendeten Guanidinderivat R und R' jeweils einen Alkylrest mit mindestens 4 Kohlenstoffatomen, einen Cycloalkylrest mit 3 bis 12 Kohlenstoffatomen oder einen carbocyclischen Arylrest mit mindestens 6 Kohlenstoffatomen bedeuten.

5. Verfahren zur Bestimmung der Sigma-Hirnrezeptor-Bindungsaktivität einer organischen Verbindung, umfassend die folgenden Schritte:
a) Inkontaktbringen in einem wäßrigen Medium einer bekannten Menge von isolierter Säugerhirnmembran, die psychotomimetische Benzomorphan-Bindungsaktivität aufweist, mit einem Gemisch aus (i) einem tritiummarkierten N,N'-disubstituierten Guanidin nach Anspruch 2, das selektiv Sigma-Hirnrezeptoren bindet, in einer Menge, von der man weiß, daß sie fähig ist, an die Sigmarezeptoren dieser Hirnmembran gebunden zu werden; und (ii) unterschiedliche bekannte Mengen einer wasserlöslichen organischen Verbindung, die auf Sigmarezeptor-Bindungsaktivität getestet werden soll;
b) Abtrennen der Hirnmembran von jeder tritiummarkierten Verbindung, die in Schritt a) nicht an die Hirnmembran gebunden wird;
c) Bestimmung der Sigmarezeptor-Bindungsaktivität der organischen Verbindung aus dem Molverhältnis des Anteils von gebundener tritiummarkierter Verbindung, die im Schritt b) abgetrennt wird, zur Molmenge der organischen Verbindung, die in Schritt a) eingesetzt wird.

6. Verfahren nach Anspruch 5, wobei die tritiummarkierte Verbindung ein disubstituiertes Guanidin der Formel 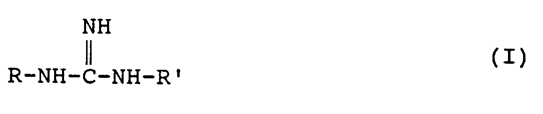 ist, wobei R und R' jeweils einen Alkylrest mit mindestens 4 Kohlenstoffatomen oder carbocyclische Arylreste mit mindestens 6 Kohlenstoffatomen bedeuten.

7. Verfahren nach Anspruch 5, wobei die tritiummarkierte Verbindung N,N'-Di-(4-[³H]-2-methylphenyl)-guanidin ist.

8. Verwendung nach Anspruch 1 oder 4, wobei das hergestellte Arzneimittel wertvoll zur Bestimmung der Beziehung von abnormalem psychoseähnlichem Verhalten in einem Säuger, der solch ein Verhalten Zeigt, zur Dysfunktion des Sigmarezeptorsystems.

9. Verwendung nach Anspruch 1 oder 4, wobei das hergestellte Arzneimittel wertvoll für die Behandlung eines Menschen ist, der an einer Psychose, die mit Halluzination verbunden ist, leidet.

10. Verwendung nach Anspruch 9, wobei das verwendete disubstituierte Guanidin eine Verbindung der Formel 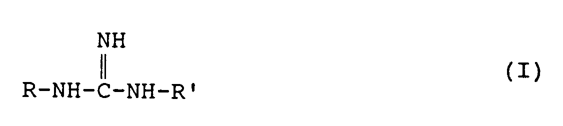 ist, in der R und R' jeweils gleich sind und einen Alkylrest mit mindestens 4 Kohlenstoffatomen, einen Cycloalkylrest mit 3 bis 12 Kohlenstoffatomen oder einen carbocyclischen Arylrest mit mindestens 6 Kohlenstoffatomen bedeuten.

11. Verbindung gemäß der Definition in Formel I nach Anspruch 1, nämlich N-Cyclohexyl-N'-(2-methylphenyl)-guanidin.

12. Verbindung gemäß der Definition in Formel I nach Anspruch 1, nämlich N-(Adamantan-1-yl)-N'-2-jodphenyl-guanidin.

13. Verbindung gemäß der Definition in Formel I nach Anspruch 1, nämlich N-(Adamantan-1-yl)-N'-(2-methylphenyl)-guanidin.

14. Arzneimittel, umfassend die Verbindung nach Anspruch 11 und einen pharmazeutisch verträglichen Träger.

15. Arzneimittel, umfassend die Verbindung nach Anspruch 12, und einen pharmazeutisch verträglichen Träger.

16. Arzneimittel, umfassend die Verbindung nach Anspruch 13 und einen pharmazeutisch verträglichen Träger.

17. Verwendung nach Anspruch 9, wobei das N,N'-disubstituierte Guanidin N-Cyclohexyl-N'-(2-methylphenyl)-guanidin ist.

18. Verwendung nach Anspruch 9, wobei das N,N'-disubstituierte Guanidin N-(Adamantan-1-yl)-N'-jodphenyl-guanidin ist.

19. Verwendung nach Anspruch 9, wobei das N,N'-disubstituierte Guanidin N-(Adamantan-1-yl)-N'-(2-methylphenyl)-guanidin ist.

20. Verwendung nach Anspruch 9, wobei das N,N'-disubstituierte Guanidin N-(Adamantan-1-yl)-N'-cyclohexyl-guanidin ist.

21. Verwendung nach Anspruch 4, wobei R eine Cyclohexylgruppe und R' eine o-Tolylgruppe ist.

22. Verwendung nach Anspruch 4, wobei R eine Adamantanylgruppe und R' eine Cyclohexylgruppe ist.

23. Verwendung nach Anspruch 4, wobei sowohl R als auch R' Adamantanylgruppen sind.

24. Verwendung nach Anspruch 4, wobei R eine Adamantanylgruppe und R' eine 2-Jodphenylgruppe ist.

25. Verwendung nach Anspruch 4, wobei R eine Adamantanylgruppe und R' eine o-Tolylgruppe ist.

## Revendications

1. Utilisation d'un dérivé de guanidine N,N'-disubstitué, de formule I 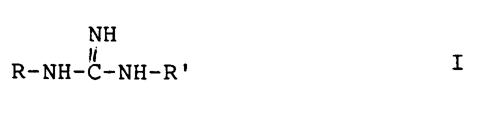 dans laquelle R et R', qui peuvent être identiques ou différents, sont des groupes alkyle ayant de 4 à 12 atomes de carbone, cycloalkyle ayant de 3 à 12 atomes de carbone, aryle carbocyclique, alkaryle ou aralkyle ayant jusqu'à 18 atomes de carbone, les groupes hydrocarbonés R et R' pouvant porter 1, 2 ou 3 des substituants suivants: des groupes alkyle ayant de 1 à 8 atomes de carbone, des atomes de chlore, brome, iode, fluor**,** nitro, azido, cyano, isocyanato, amino, (alkyl inférieur)-amino, di-(alkyl inférieur)-amino, trifluorométhyle, alcoxy ayant de 1 à 8 atomes de carbone, acyloxy ayant de 1 à 8 atomes de carbone, amido, N-éthylacétamido, carbamido, N-méthylcarbamoyle et N,N'-diméthylcarbamoyle, ou de la N-(2-méthyl-4-isothiocyanatophényl)-N'-(2-méthylphényl)-guanidine, pour la préparation d'une composition pharmaceutique utile pour le diagnostic et le traitement de psychoses.

2. Dérivé de guanidine N,N'-disubstitué de formule I, tel que décrit dans la revendication 1 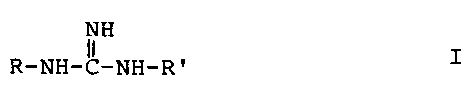 formule dans laquelle R et R' sont chacun un groupe alkyle ayant au moins 4 atomes de carbone, adamantyle, cyclohexyle ou un groupe aryle carbocyclique ayant au moins 6 atomes de carbone et dans lequel au moins l'un des atomes de carbone formant le cycle de R et R' porte au moins un atome de tritium.

3. Dérivé selon la revendication 2, qui est la N,N'-di-(4-[³H]-2-méthylphényl)-guanidine.

4. Utilisation selon la revendication 1, dans laquelle, dans le dérivé de guanidine utilisé, R et R' sont chacun un groupe alkyle ayant au moins 4 atomes de carbone, un groupe cycloalkyle ayant de 3 à 12 atomes de carbone ou un groupe aryle carbocyclique ayant au moins 6 atomes de carbone.

5. Procédé pour la détermination de l'activité de liaison aux récepteurs cérébraux sigma, manifestée par un composé organique, comprenant les étapes suivantes:
a) mise en contact, dans un milieu aqueux, d'une quantité connue de membrane cérébrale mammalienne isolée, ayant une activité de liaison au psychotomimétique benzomorphan, avec un mélange de (I) une guanidine N,N'-disubstituée marquée au tritium, selon la revendication 2, qui se lie sélectivement aux récepteurs cérébraux sigma, en une quantité connue capable d'être liée aux récepteurs cérébraux sigma; et de (II) diverses quantités connues d'un compose organique soluble dans l'eau, à soumettre à un essai de l'activité de liaison aux récepteurs sigma;
b) séparation de la membrane cérébrale d'avec l'un quelconque des composés marqués au tritium qui n'est pas lié à la membrane cérébrale dans l'étape a);
c) détermination, d'après le rapport molaire, de la proportion de composé lié, marqué au tritium, qui est séparé dans l'étape b), à la quantité molaire du composé organique utilisé dans l'étape a), de l'activité de liaison aux récepteurs sigma qui est manifestée par ce composé organique.

6. Procédé selon la revendication 5, dans lequel le composé marqué au tritium est une guanidine disubstituée de formule 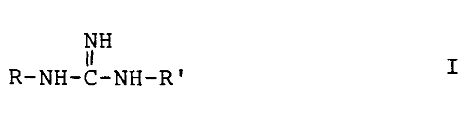 dans laquelle R et R' sont chacun un groupe alkyle ayant au moins 4 atomes de carbone ou un groupe aryle carbocyclique ayant au moins 6 atomes de carbone.

7. Procédé selon la revendication 5, dans lequel le composé marqué au tritium est la N,N'-di-(4-[³H]-2-méthylphényl)guanidine.

8. Utilisation selon la revendication 1 ou 4, dans laquelle la composition pharmaceutique préparée est utile dans la détermination de la relation d'un comportement anormal de type psychotique, chez un mammifère faisant preuve d'un tel comportement, avec une dysfonction du système des récepteurs sigma.

9. Utilisation selon la revendication 1 ou 4, dans laquelle la composition pharmaceutique préparée est utile dans le traitement d'un sujet humain souffrant d'une maladie mentale psychotique associée à des hallucinations.

10. Utilisation selon la revendication 9, dans laquelle la guanidine disubstituée utilisée est un composé de formule 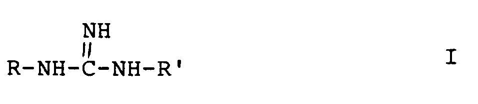 dans laquelle R et R' sont identiques et chacun est un groupe alkyle ayant au moins 4 atomes de carbone, un groupe cycloalkyle ayant de 3 à 12 atomes ou un groupe aryle carbocyclique ayant au moins 6 atomes de carbone.

11. Composé tel que défini dans la formule I de la revendication 1, à savoir la N-cyclohexyl-N'-(2-méthylphényl)-guanidine.

12. Composé tel que défini dans la formule I de la revendication 1, à savoir la N-(adamantan-1-yl)-N'-2-iodophényl-guanidine.

13. Composé tel que défini dans la formule I de la revendication 1, à savoir la N-(adamantan-1-yl)-N'-(2-méthylphényl)-guanidine.

14. Composition pharmaceutique comprenant le compose de la revendication 11 et un véhicule pharmaceutiquement acceptable.

15. Composition pharmaceutique comprenant le compose de la revendication 12 et un véhicule pharmaceutiquement acceptable.

16. Composition pharmaceutique comprenant le compose de la revendication 13 et un véhicule pharmaceutiquement acceptable.

17. Utilisation selon la revendication 9, dans laquelle ladite guanidine N,N'-disubstituée est la N-cyclohexyl-N'-(2-méthylphényl)-guanidine.

18. Utilisation selon la revendication 9, dans laquelle ladite guanidine N,N'-disubstituée est la N-(adamantan-1-yl)-N'-iodophényl-guanidine.

19. Utilisation selon la revendication 9, dans laquelle ladite guanidine N,N'-disubstituée est la N-(adamantan-1-yl)-N'-(2-méthylphényl)-guanidine.

20. Utilisation selon la revendication 9, dans laquelle ladite guanidine N,N'-disubstituée est la N-(adamantan-1-yl)-N'-cyclohexyl-guanidine.

21. Utilisation selon la revendication 4, dans laquelle R est le groupe cyclohexyle et R' est le groupe o-tolyle.

22. Utilisation selon la revendication 4, dans laquelle R est le groupe adamantanyle et R' est le groupe cyclohexyle.

23. Utilisation selon la revendication 4, dans laquelle R et R' sont l'un et l'autre le groupe adamantanyle.

24. Utilisation selon la revendication 4, dans laquelle R est le groupe adamantanyle et R' est le groupe 2-iodophényle.

25. Utilisation selon la revendication 4, dans laquelle R est le groupe adamantanyle et R' est le groupe o-tolyle.
